# EUROPÄISCHE PATENTSCHRIFT

(11) **EP 1 572 993 B1**
(45) Veröffentlichungstag und Bekanntmachung des Hinweises auf die Patenterteilung: **03.02.2010**
(21) Anmeldenummer: 03767793.7
(22) Anmeldetag: 12.12.2003
(51) Int. Cl.: C12N 9/04, C12N 15/52, A61Q 5/10, D06L 3/11

(54) **CHOLINOXIDASEN**
CHOLINE OXIDASES
CHOLINES OXYDASES

(30) Priorität: 20.12.2002 DE 10260930
(43) Veröffentlichungstag der Anmeldung: 14.09.2005
(73) Patentinhaber: Henkel AG & Co. KGaA, 40589 Düsseldorf (DE)
(72) Erfinder: SAUTER, Kerstin, 40217 Düsseldorf (DE); WEISS, Albrecht, 40764 Langenfeld (DE); MAURER, Karl-Heinz, 40699 Erkrath (DE); EVERS, Stefan, 40822 Mettmann (DE); HOVEN, Nina, 41061 Mönchengladbach (DE); WIELAND, Susanne, 41541 Dormagen-Zons (DE); STEHR, Regina, 41468 Neuss (DE); BESSLER, Cornelius, 40597 Düsseldorf (DE)
(86) Internationale Anmeldenummer: PCT/EP2003/014130
(87) Internationale Veröffentlichungsnummer: WO 2004/058955

(56) Entgegenhaltungen:
- EP-A- 0 818 138
- WO-A-97/21796
- WO-A-99/37786
- DESHNIUM P ET AL: "TRANSFORMATION OF SYNECHOCOCCUS WITH A GENE FOR CHOLINE OXIDASE ENHANCES TOLERANCE TO SALT STRESS" PLANT MOLECULAR BIOLOGY, NIJHOFF PUBLISHERS, DORDRECHT, NL, Bd. 29, Nr. 5, Dezember 1995 (1995-12), Seiten 897-907, XP000942466 ISSN: 0167-4412 in der Anmeldung erwähnt

### Sequenzprotokoll - SEQUENCE LISTING

<110> Henkel Kommanditgesellschaft auf Aktien
<120> Neue Cholinoxidasen
<130> H 05753 PCT
<150> 102 60 930.6-41
   <151> 2002-12-20
<160> 24
<170> SeqWin99, version 1.02
<210> 1
   <211> 542
   <212> PRT
   <213> Arthrobacter nicotinianae
<220>
   <223> Cholinoxidase aus Arthrobacter nicotianiae (KC2, DSMZ-ID 96-878)
<400> 1
   Thr Arg Lys Gly Glu Lys Leu Asn Ile Glu Lys Lys Asp Phe Asp Tyr
   1               5                   10                  15
   Ile Val Ile Gly Gly Gly Ser Ala Gly Ala Ala Val Ala Ser Arg Leu
               20                  25                  30
   Ser Glu Asp Pro Ser Val Ser Val Ala Leu Val Glu Ala Gly Pro Asp
           35                  40                  45
   Asp Arg Gly Tyr Asp Glu Val Leu Gln Leu Asp Arg Trp Met Glu Leu
       50                  55                  60
   Leu Glu Ser Gly Leu Asp Trp Asp Tyr Pro Ile Glu Pro Gln Glu Asn
   65                  70                  75                  80
   Gly Asn Ser Phe Met Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Phe Gly Ala Thr Gly Trp Asn Ser Glu Met
           115                 120                 125
   Ala Tyr Arg Leu Tyr Lys Lys Leu Glu Thr Asn Glu Asp Ala Gly Pro
       130                 135                 140
   Asp Ala Pro His His Gly Asp Ser Gly Pro Val Lys Leu Met Asn Val
   145                 150                 155                 160
   Pro Pro Val Asp Pro Cys Gly Val Ala Ile Leu Asp Ala Ala Glu Gln
                   165                 170                 175
   Ala Gly Ile Pro Arg Ala Lys Phe Asn Asn Asn Glu Thr Val Ile Asn
               180                 185                 190
   Gly Ala Asn Phe Phe Gln Ile Asn Arg Leu Pro Asp Gly Thr Arg Ala
           195                 200                 205
   Ser Ser Ser Val Ser Tyr Ile His Pro Ile Glu Gly Arg Glu Asn Phe
   210 215 220
   Phe Leu Leu Thr Gly Leu Gln Ala Arg Lys Leu Asn Phe Asp Ala Asp
   225 230 235 240
   Lys Arg Cys Thr Gly Val Asp Val Val Asp Gly Ala Phe Gly Arg Thr
   245 250 255
   Val Thr Leu Asn Ala Ala Arg Glu Val Val Val Ser Ala Gly Ala Ile
   260 265 270
   Asp Ser Pro Lys Leu Leu Met Leu Ser Gly Ile Gly Pro Ala Glu His
   275 280 285
   Leu Lys Glu Val Gly Val Glu Val Leu Val Asp Ala Pro Gly Val Gly
   290 295 300
   Glu His Leu Gln Asp His Pro Glu Gly Val Ile Gln Trp Glu Ala Lys
   305 310 315 320
   Lys Pro Met Val Glu Thr Ser Thr Gln Trp Trp Glu Ile Gly Ile Phe
   325 330 335
   Ala Pro Thr Gln Glu Gly Leu Asp Arg Pro Asp Leu Met Met His Tyr
   340 345 350
   Gly Ser Val Pro Phe Asp Met His Thr Leu Arg Trp Gly Tyr Pro Thr
   355 360 365
   Ser Glu Asn Thr Phe Cys Leu Thr Pro Asn Val Thr His Ala Lys Ser
   370 375 380
   Arg Gly Thr Val Arg Leu Arg Ser Cys Asp Phe Ser Asp Lys Pro Lys
   385 390 395 400
   Val Asp Pro Arg Tyr Phe Thr Asp Pro Glu Gly His Asp Ala Arg Val
   405 410 415
   Met Thr Phe Gly Ile Arg Lys Ala Arg Glu Ile Val Ala Gln Ser Pro
   420 425 430
   Met Ala Glu Trp Ala Gly Glu Glu Gln Phe Pro Gly Lys Asp Val Gln
   435 440 445
   Thr Asp Glu Glu Ile Phe Asp Tyr Leu Arg Arg Cys His Asn Thr Val
   450 455 460
   Tyr His Pro Ala Gly Ser Val Arg Met Gly Ala Glu Asp Asp Val Met
   465 470 475 480
   Ser Pro Leu Asp Pro Gln Leu Arg Val Lys Gly Val Ser Gly Leu Arg
   485 490 495
   Val Ala Asp Ala Ser Val Met Pro Glu Leu Val Thr Val Asn Pro Asn
   500 505 510
   Ile Thr Val Met Met Ile Gly Glu Arg Cys Ala Glu Leu Ile Gln Glu
   515 520 525
   Asn Pro Val Glu Ala Arg Gln Ser Glu Leu Val Ser Glu Ala
   530 535 540
<210> 2
   <211> 550
   <212> PRT
   <213> Arthrobacter aurescens
<220>
   <223> Cholinoxidase aus Arthrobacter aurescens (ATCC 13344)
<400> 2
   Met His Val Asp Asn Ile Glu Asn Leu Ser Glu Arg Gly Phe Asp Tyr
   1               5                   10                  15
   Ile Val Ile Gly Gly Gly Ser Ala Gly Ala Ala Val Ala Ala Arg Leu
               20                  25                  30
   Ser Glu Asp Pro Ala Val Thr Val Ala Leu Val Glu Ala Gly Pro Asp
           35                  40                  45
   Asp Arg Asn Leu Pro Glu Ile Leu Gln Leu Asp Arg Trp Met Glu Leu
       50                  55                  60
   Leu Glu Ser Gly Tyr Asp Trp Asp Tyr Pro Val Glu Pro Gln Glu Asn
   65                  70                  75                  80
   Gly Asn Ser Phe Met Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Tyr Gly Ala Thr Gly Trp Asn Ala Ala Ala
           115                 120                 125
   Ala Trp Pro Leu Tyr Gln Arg Leu Glu Ser Asn Glu Asp Ala Gly Pro
       130                 135                 140
   Asp Ala Pro His His Gly His Asp Gly Pro Val His Leu Met Asn Val
   145                 150                 155                 160
   Pro Pro Asn Asp Pro Ala Gly Val Ala Leu Leu Asp Ala Cys Glu Gln
                   165                 170                 175
   Ser Gly Ile Pro Arg Ala Lys Phe Asn Asp Gly Thr Thr Val Ile Asn
               180                 185                 190
   Gly Ala Asn Phe Phe Gln Ile Asn Arg Arg Ser Asp Gly Thr Arg Ser
           195                 200                 205
   Ser Ser Ser Val Ser Tyr Ile His Pro Ile Met Ala Arg Glu Asn Phe
       210                 215                 220
   Thr Leu Leu Thr Gly Leu Arg Ala Arg Gln Leu Val Phe Asp Ala Asp
   225                 230                 235                 240
   Lys Arg Cys Thr Gly Val Asp Val Val Asp Ser Ala Phe Gly Arg Thr
                   245                 250                 255
   His Arg Leu Thr Ala Gln Arg Glu Val Ile Leu Ser Thr Gly Ala Ile
               260                 265                 270
   Asp Ser Pro Lys Leu Leu Met Leu Ser Gly Ile Gly Pro Ala Glu His
           275                 280                 285
   Leu Ala Gln His Gly Ile Glu Val Val Val Asp Ser Pro Gly Val Gly
   290 295 300
   Glu His Leu Gln Asp His Pro Glu Gly Val Val Gln Phe Glu Ala Lys
   305 310 315 320
   Gln Pro Met Val Gln Ser Ser Thr Gln Trp Trp Glu Ile Gly Ile Phe
   325 330 335
   Thr Pro Thr Glu Glu Gly Leu Asp Arg Pro Asp Leu Met Met His Tyr
   340 345 350
   Gly Ser Val Pro Phe Asp Met Asn Thr Leu Arg Tyr Gly Tyr Pro Thr
   355 360 365
   Thr Glu Asn Gly Phe Ser Leu Thr Pro Asn Val Thr His Ala Arg Ser
   370 375 380
   Arg Gly Thr Val Arg Leu Arg Ser Arg Asp Phe Arg Asp Lys Pro Met
   385 390 395 400
   Val Asp Pro Arg Tyr Phe Thr Asp Pro Glu Gly His Asp Met Arg Val
   405 410 415
   Met Val Ala Gly Ile Arg Lys Ala Arg Glu Ile Ala Ala Gln Pro Ala
   420 425 430
   Met Ala Glu Trp Thr Gly Arg Glu Leu Ser Pro Gly Ile Glu Ala Gln
   435 440 445
   Thr Asp Glu Glu Leu Gln Asp Tyr Ile Arg Lys Thr His Asn Thr Val
   450 455 460
   Tyr His Pro Val Gly Thr Val Arg Met Gly Pro Val Glu Asp Pro Met
   465 470 475 480
   Ser Pro Leu Asp Pro Glu Leu Arg Val Lys Gly Val Ser Gly Leu Arg
   485 490 495
   Val Ala Asp Ala Ser Val Met Pro Glu His Val Thr Val Asn Pro Asn
   500 505 510
   Ile Thr Val Met Met Ile Gly Glu Arg Cys Ala Asp Leu Ile Lys Ala
   515 520 525
   Arg Leu Gly Ala Ser Arg Val Glu Glu Thr Thr Thr Thr Glu Ala Asp
   530 535 540
   Phe Ser Leu Ser His Ala
   545 550
<210> 3
   <211> 550
   <212> PRT
   <213> Artificial Sequence
<220>
   <223> Hybride Cholinoxidase
<400> 3
   Met His Val Asp Asn Ile Glu Asn Leu Ser Glu Arg Gly Phe Asp Tyr
   1 5 10 15
   Ile Val Ile Gly Gly Gly Ser Ala Gly Ala Ala Val Ala Ala Arg Leu
               20                  25                  30
   Ser Glu Asp Pro Ala Val Thr Val Ala Leu Val Glu Ala Gly Pro Asp
           35                  40                  45
   Asp Arg Asn Leu Pro Glu Ile Leu Gln Leu Asp Arg Trp Met Glu Leu
       50                  55                  60
   Leu Glu Ser Gly Tyr Asp Trp Asp Tyr Pro Val Glu Pro Gln Glu Asn
   65                  70                  75                  80
   Gly Asn Ser Phe Met Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Phe Gly Ala Thr Gly Trp Asn Ser Glu Met
           115                 120                 125
   Ala Tyr Arg Leu Tyr Lys Lys Leu Glu Thr Asn Glu Asp Ala Gly Pro
       130                 135                 140
   Asp Ala Pro His His Gly Asp Ser Gly Pro Val Lys Leu Met Asn Val
   145                 150                 155                 160
   Pro Pro Val Asp Pro Cys Gly Val Ala Ile Leu Asp Ala Ala Glu Gln
                   165                 170                 175
   Ala Gly Ile Pro Arg Ala Lys Phe Asn Asn Asn Glu Thr Val Ile Asn
               180                 185                 190
   Gly Ala Asn Phe Phe Gln Ile Asn Arg Leu Pro Asp Gly Thr Arg Ala
           195                 200                 205
   Ser Ser Ser Val Ser Tyr Ile His Pro Ile Glu Gly Arg Glu Asn Phe
       210                 215                 220
   Phe Leu Leu Thr Gly Leu Gln Ala Arg Lys Leu Asn Phe Asp Ala Asp
   225                 230                 235                 240
   Lys Arg Cys Thr Gly Val Asp Val Val Asp Gly Ala Phe Gly Arg Thr
                   245                 250                 255
   Val Thr Leu Asn Ala Ala Arg Glu Val Val Val Ser Ala Gly Ala Ile
               260                 265                 270
   Asp Ser Pro Lys Leu Leu Met Leu Ser Gly Ile Gly Pro Ala Glu His
           275                 280                 285
   Leu Lys Glu Val Gly Val Glu Val Leu Val Asp Ala Pro Gly Val Gly
       290                 295                 300
   Glu His Leu Gln Asp His Pro Glu Gly Val Ile Gln Trp Glu Ala Lys
   305                 310                 315                 320
   Lys Pro Met Val Glu Thr Ser Thr Gln Trp Trp Glu Ile Gly Ile Phe
                   325                 330                 335
   Ala Pro Thr Gln Glu Gly Leu Asp Arg Pro Asp Leu Met Met His Tyr
               340                 345                 350
   Gly Ser Val Pro Phe Asp Met His Thr Leu Arg Trp Gly Tyr Pro Thr
   355 360 365
   Ser Glu Asn Thr Phe Cys Leu Thr Pro Asn Val Thr His Ala Lys Ser
   370 375 380
   Arg Gly Thr Val Arg Leu Arg Ser Arg Asp Phe Arg Asp Lys Pro Met
   385 390 395 400
   Val Asp Pro Arg Tyr Phe Thr Asp Pro Glu Gly His Asp Met Arg Val
   405 410 415
   Met Val Ala Gly Ile Arg Lys Ala Arg Glu Ile Ala Ala Gln Pro Ala
   420 425 430
   Met Ala Glu Trp Thr Gly Arg Glu Leu Ser Pro Gly Ile Glu Ala Gln
   435 440 445
   Thr Asp Glu Glu Leu Gln Asp Tyr Ile Arg Lys Thr His Asn Thr Val
   450 455 460
   Tyr His Pro Val Gly Thr Val Arg Met Gly Pro Val Glu Asp Pro Met
   465 470 475 480
   Ser Pro Leu Asp Pro Glu Leu Arg Val Lys Gly Val Ser Gly Leu Arg
   485 490 495
   Val Ala Asp Ala Ser Val Met Pro Glu His Val Thr Val Asn Pro Asn
   500 505 510
   Ile Thr Val Met Met Ile Gly Glu Arg Cys Ala Asp Leu Ile Lys Ala
   515 520 525
   Arg Leu Gly Ala Ser Arg Val Glu Glu Thr Thr Thr Thr Glu Ala Asp
   530 535 540
   Phe Ser Leu Ser His Ala
   545 550
<210> 4
   <211> 1629
   <212> DNA
   <213> Arthrobacter nicotianiae
<220>
   <223> Cholinoxidase aus Arthrobacter nicotianiae (KC2, DSMZ-ID 96-878)
<400> 4
   acgagaaagg gcgaaaaatt gaacattgaa aagaaggact tcgactacat tgtgatcggc    60
   ggcggttctg ccggcgctgc ggtggcttct cgcctgagcg aggatccatc ggtgagtgtt   120
   gcactggttg aagcaggtcc cgacgaccgc ggctacgatg aagtgctgca gctggaccgc   180
   tggatggaac tgctggaatc cggcttggac tgggactacc cgatcgaacc gcaggaaaac   240
   ggcaactcgt tcatgcgcca tgcccgtgcc aaggtcatgg gcggctgctc cagccacaac   300
   tcctgcatcg ccttctgggc gccgcgtgag gaccttgacg agtgggagtc gaagttcggc   360
   gccaccggct ggaactccga gatggcctac cgcctgtaca agaagctgga aaccaatgag   420
   gacgccggac cggatgcacc gcaccacggc gactcgggcc cggtcaagct gatgaacgtg   480
   ccgccggtgg acccctgcgg cgtggcaatc ctggacgccg ccgagcaggc aggcatcccg   540
   cgcgccaagt tcaacaacaa cgagacggtc atcaacggcg cgaacttctt ccagatcaac   600
   cgcctgccgg acggcacccg tgcctcctcc tcggtctcct acatccaccc gatcgaggga   660
   cgcgagaact tcttcctgct caccggactg caggcgcgca agctgaactt cgacgccgac   720
   aagcgctgca ccggcgtgga cgtggtggac ggggccttcg gccgcaccgt gacgctgaac   780
   gccgcccgcg aggtcgtcgt ctccgccggc gccattgact cgccgaagct gctgatgctc   840
   tccggcatcg gacctgccga acacctcaag gaggtcggcg tcgaggtgct tgtcgacgct   900
   ccaggcgtgg gcgaacacct gcaggaccac ccggaaggcg tgatccagtg ggaggctaag   960
   aagccgatgg ttgaaacctc cacccagtgg tgggaaatcg gcatcttcgc accgacccag  1020
   gaaggcctcg accgtccgga tctgatgatg cactacggtt cggtgccctt cgacatgcac  1080
   accctgcgtt ggggttaccc gaccagcgag aacaccttct gcctgacccc gaatgtcacc  1140
   catgcgaagt cgcgcggcac cgtgcgcctg cgcagctgcg acttcagcga caagccgaag  1200
   gtcgaccctc gctacttcac cgatccagag ggccacgacg cccgggtcat gaccttcggc  1260
   atccgcaagg cccgcgagat cgtcgcccag tcgccgatgg cagagtgggc tggcgaggag  1320
   cagttcccgg gcaaggacgt gcagactgac gaagagatct tcgactacct gcgccgctgc  1380
   cacaacactg tctaccaccc ggctggttcg gtgcgcatgg gcgccgagga cgatgtgatg  1440
   agcccgctgg atccgcagct gcgggtcaag ggagtctcgg gcctgcgcgt ggccgacgct  1500
   tcggtgatgc cggagctggt caccgtgaac ccgaacatca cggtgatgat gatcggtgaa  1560
   cgctgcgccg agctgattca ggaaaaccct gtggaagccc gccagagcga actggttagc  1620
   gaagcctag                                                          1629
<210> 5
   <211> 1650
   <212> DNA
   <213> Arthrobacter aurescens
<220>
   <223> Cholinoxidase aus Arthrobacter aurescens ATCC 13344
<400> 5
   atgcacgtcg acaatattga gaacctcagc gagcgcgggt tcgattacat cgtcatcggc   60
   ggaggatctg ccggggctgc cgtcgccgcc cggctgagcg aggaccccgc cgttaccgtt  120
   gccctggtgg aagccggccc ggatgaccgg aacctcccgg aaatcctgca attggaccgg  180
   tggatggaac tgctggaatc ggggtacgac tgggactacc cggtggaacc gcaggagaac  240
   ggcaactcat tcatgcgcca cgcccgcgcc aaggtgatgg gcggctgttc cagccacaat  300
   tcctgcatcg ctttctgggc tccccgggag gacctggatg agtgggagtc caagtacggt  360
   gccaccggtt ggaacgctgc cgcggcctgg cctttgtatc agcgcttgga aagcaacgag  420
   gacgctggcc cggatgcccc acatcacggc catgatggtc cggtgcacct gatgaacgtt  480
   cctccgaatg atccggccgg ggttgcgctc ctggacgcct gtgagcagag cggcattccc  540
   cgggcaaagt tcaacgacgg caccacagtg atcaacggcg ctaatttctt ccagatcaac  600
   cgccgttcgg acggtacgcg ttcctccagt tctgtttcct acattcaccc catcatggct  660
   cgcgaaaact tcacgctgtt gaccggcctg cgtgcccgcc aactggtgtt cgacgcggac  720
   aagcgttgca ccggcgtcga cgtcgttgac tctgccttcg gcaggacaca caggctcacc  780
   gcgcagcgcg aggtcatcct ctccacgggc gccattgatt cccccaagct gctcatgctt  840
   tccggaatcg gcccggccga gcacctggct caacatggca tcgaggtggt ggtggactcc  900
   cccggtgttg gcgagcatct gcaggaccac ccggagggcg tggtgcagtt cgaggccaag  960
   cagcccatgg ttcagtcgtc cacccaatgg tgggagatcg gcatcttcac tcccaccgag 1020
   gagggactgg accgccccga cctgatgatg cactacggct ccgtgccgtt cgacatgaac 1080
   accctccgct acggttaccc caccacggag aacggcttca gccttacccc gaacgtcacg 1140
   cacgcccgtt cccgcgggac cgtccgtttg cgcagcaggg acttccgcga caagcccatg 1200
   gtggacccgc ggtacttcac cgatcccgag gggcacgaca tgcgcgtcat ggtggccggc 1260
   atccgcaagg cccgggaaat cgcggctcag ccggccatgg ccgagtggac cggacgggag 1320
   ctctcccccg gaatcgaggc ccagactgat gaggagcttc aggactacat ccgcaaaacc 1380
   cacaacaccg tctaccaccc cgtcggtacc gtccgcatgg gcccggtgga ggaccccatg 1440
   tctcccttgg atccggagct gcgtgtcaaa ggcgtcagcg gcctacgggt ggcagacgcc 1500
   tccgtgatgc ccgaacacgt cacagtgaac cccaacatca ccgtgatgat gatcggcgag 1560
   cgctgtgctg atctcatcaa agcccggctg ggtgccagcc gggtggagga aaccacgacg 1620
   acggaggcgg acttcagctt gtcccacgcc                                 1650
<210> 6
   <211> 1650
   <212> DNA
   <213> Artificial Sequence
<220>
   <223> hybride Cholinoxidase
<400> 6
   atgcacgtcg acaatattga gaacctcagc gagcgcgggt tcgattacat cgtcatcggc   60
   ggaggatctg ccggggctgc cgtcgccgcc cggctgagcg aggaccccgc cgttaccgtt  120
   gccctggtgg aagccggccc ggatgaccgg aacctcccgg aaatcctgca attggaccgg   180
   tggatggaac tgctggaatc ggggtacgac tgggactacc cggtggaacc gcaggagaac   240
   ggcaactcat tcatgcgcca cgcccgcgcc aaggtcatgg gcggctgctc cagccacaac   300
   tcctgcatcg ccttctgggc gccgcgtgag gaccttgacg agtgggagtc gaagttcggc   360
   gccaccggct ggaactccga gatggcctac cgcctgtaca agaagctgga aaccaatgag   420
   gacgccggac cggatgcacc gcaccacggc gactcgggcc cggtcaagct gatgaacgtg   480
   ccgccggtgg acccctgcgg cgtggcaatc ctggacgccg ccgagcaggc aggcatcccg   540
   cgcgccaagt tcaacaacaa cgagacggtc atcaacggcg cgaacttctt ccagatcaac   600
   cgcctgccgg acggcacccg tgcctcctcc tcggtctcct acatccaccc gatcgaggga   660
   cgcgagaact tcttcctgct caccggactg caggcgcgca agctgaactt cgacgccgac   720
   aagcgctgca ccggcgtgga cgtggtggac ggggccttcg gccgcaccgt gacgctgaac   780
   gccgcccgcg aggtcgtcgt ctccgccggc gccattgact cgccgaagct gctgatgctc   840
   tccggcatcg gacctgccga acacctcaag gaggtcggcg tcgaggtgct tgtcgacgct   900
   ccaggcgtgg gcgaacacct gcaggaccac ccggaaggcg tgatccagtg ggaggctaag   960
   aagccgatgg ttgaaacctc cacccagtgg tgggaaatcg gcatcttcgc accgacccag  1020
   gaaggcctcg accgtccgga tctgatgatg cactacggtt cggtgccctt cgacatgcac  1080
   accctgcgtt ggggttaccc gaccagcgag aacaccttct gcctgacccc gaatgtcacc  1140
   catgcgaagt cgcgcggcac cgtgcgcctg cgcagcaggg acttccgcga caagcccatg  1200
   gtggacccgc ggtacttcac cgatcccgag ggcacgacaa tgcgcgtcat ggtggccggc  1260
   atccgcaagg cccgggaaat cgcggctcag ccggccatgg ccgagtggac cggacgggag  1320
   ctctcccccg gaatcgaggc ccagactgat gaggagcttc aggactacat ccgcaaaacc  1380
   cacaacaccg tctaccaccc cgtcggtacc gtccgcatgg gcccggtgga ggaccccatg  1440
   tctcccttgg atccggagct gcgtgtcaaa ggcgtcagcg gcctacgggt ggcagacgcc  1500
   tccgtgatgc ccgaacacgt cacagtgaac cccaacatca ccgtgatgat gatcggcgag  1560
   cgctgtgctg atctcatcaa agcccggctg ggtgccagcc gggtggagga aaccacgacg  1620
   acggaggcgg acttcagctt gtcccacgcc                                    1650
<210> 7
   <211> 902
   <212> DNA
   <213> Artificial Sequence
<220>
   <223> Sonde zur Isolierung einer Cholinoxidase
<400> 7
   caaggtcatg ggcggctgct ccagccacaa ctcctgcatc gccttctggg cgccgcgtga   60
   ggaccttgac gagtgggagt cgaagttcgg cgccaccggc tggaactccg agatggccta  120
   ccgcctgtac aagaagctgg aaaccaatga ggacgccgga ccggatgcac cgcaccacgg  180
   cgactcgggc ccggtcaagc tgatgaacgt gccgccggtg gacccctgcg gcgtggcaat  240
   cctggacgcc gccgagcagg caggcatccc gcgcgccaag ttcaacaaca acgagacggt  300
   catcaacggc gcgaacttct tccagatcaa ccgcctgccg gacggcaccc gtgcctcctc  360
   ctcggtctcc tacatccacc cgatcgaggg acgcgagaac ttcttcctgc tcaccggact  420
   gcaggcgcgc aagctgaact tcgacgccga caagcgctgc accggcgtgg acgtggtgga  480
   cggggccttc ggccgcaccg tgacgctgaa cgccgcccgc gaggtcgtcg tctccgccgg  540
   cgccattgac tcgccgaagc tgctgatgct ctccggcatc ggacctgccg aacacctcaa  600
   ggaggtcggc gtcgaggtgc ttgtcgacgc tccaggcgtg ggcgaacacc tgcaggacca  660
   cccggaaggc gtgatccagt gggaggctaa gaagccgatg gttgaaacct ccacccagtg  720
   gtgggaaatc ggcatcttcg caccgaccca ggaaggcctc gaccgtccgg atctgatgat  780
   gcactacggt tcggtgccct tcgacatgca caccctgcgt tggggttacc cgaccagcga  840
   gaacaccttc tgcctgaccc cgaatgtcac ccatgcgaag tcgcgcggca ccgtgcgcct  900
   gc                                                                  902
<210> 8
   <211> 34
   <212> DNA
   <213> Artificial Sequence
<220>
   <223> Primer zur Klonierung einer Cholinoxidase
<400> 8
   catgccatgg caaagggcga aaaattgaac attg 34
<210> 9
   <211> 31
   <212> DNA
   <213> Artificial Sequence
<220>
   <223> Primer zur Klonierung einer Cholinoxidase
<400> 9
   cccaagcttc taggcttcgc taaccagttc g 31
<210> 10
   <211> 542
   <212> PRT
   <213> Artificial Sequence
<220>
   <223> Expreasionsprodukt von Nukleinsäuresequenz KC2 in E. coli
<400> 10
   Met Ala Lys Gly Glu Lys Leu Asn Ile Glu Lys Lys Asp Phe Asp Tyr
   1               5                   10                  15
   Ile Val Ile Gly Gly Gly Ser Ala Gly Ala Ala Val Ala Ser Arg Leu
               20                  25                  30
   Ser Glu Asp Pro Ser Val Ser Val Ala Leu Val Glu Ala Gly Pro Asp
           35                  40                  45
   Asp Arg Gly Tyr Asp Glu Val Leu Gln Leu Asp Arg Trp Met Glu Leu
       50                  55                  60
   Leu Glu Ser Gly Leu Asp Trp Asp Tyr Pro Ile Glu Pro Gln Glu Asn
   65                  70                  75                  80
   Gly Asn Ser Phe Met Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Phe Gly Ala Thr Gly Trp Asn Ser Glu Met
           115                 120                 125
   Ala Tyr Arg Leu Tyr Lys Lys Leu Glu Thr Asn Glu Asp Ala Gly Pro
       130                 135                 140
   Asp Ala Pro His His Gly Asp Ser Gly Pro Val Lys Leu Met Asn Val
   145                 150                 155                 160
   Pro Pro Val Asp Pro Cys Gly Val Ala Ile Leu Asp Ala Ala Glu Gln
                   165                 170                 175
   Ala Gly Ile Pro Arg Ala Lys Phe Asn Asn Asn Glu Thr Val Ile Asn
               180                 185                 190
   Gly Ala Asn Phe Phe Gln Ile Asn Arg Leu Pro Asp Gly Thr Arg Ala
           195                 200                 205
   Ser Ser Ser Val Ser Tyr Ile His Pro Ile Glu Gly Arg Glu Asn Phe
       210                 215                 220
   Phe Leu Leu Thr Gly Leu Gln Ala Arg Lys Leu Asn Phe Asp Ala Asp
   225                 230                 235                 240
   Lys Arg Cys Thr Gly Val Asp Val Val Asp Gly Ala Phe Gly Arg Thr
                   245                 250                 255
   Val Thr Leu Asn Ala Ala Arg Glu Val Val Val Ser Ala Gly Ala Ile
               260                 265                 270
   Asp Ser Pro Lys Leu Leu Met Leu Ser Gly Ile Gly Pro Ala Glu His
           275                 280                 285
   Leu Lys Glu Val Gly Val Glu Val Leu Val Asp Ala Pro Gly Val Gly
       290                 295                 300
   Glu His Leu Gln Asp His Pro Glu Gly Val Ile Gln Trp Glu Ala Lys
   305                 310                 315                 320
   Lys Pro Met Val Glu Thr Ser Thr Gln Trp Trp Glu Ile Gly Ile Phe
                   325                 330                 335
   Ala Pro Thr Gln Glu Gly Leu Asp Arg Pro Asp Leu Met Met His Tyr
               340                 345                 350
   Gly Ser Val Pro Phe Asp Met His Thr Leu Arg Trp Gly Tyr Pro Thr
           355                 360                 365
   Ser Glu Asn Thr Phe Cys Leu Thr Pro Asn Val Thr His Ala Lys Ser
       370                 375                 380
   Arg Gly Thr Val Arg Leu Arg Ser Cys Asp Phe Ser Asp Lys Pro Lys
   385                 390                 395                 400
   Val Asp Pro Arg Tyr Phe Thr Asp Pro Glu Gly His Asp Ala Arg Val
                   405                 410                 415
   Met Thr Phe Gly Ile Arg Lys Ala Arg Glu Ile Val Ala Gln Ser Pro
               420                 425                 430
   Met Ala Glu Trp Ala Gly Glu Glu Gln Phe Pro Gly Lys Asp Val Gln
           435                 440                 445
   Thr Asp Glu Glu Ile Phe Asp Tyr Leu Arg Arg Cys His Asn Thr Val
       450                 455                 460
   Tyr His Pro Ala Gly Ser Val Arg Met Gly Ala Glu Asp Asp Val Met
   465                 470                 475                 480
   Ser Pro Leu Asp Pro Gln Leu Arg Val Lys Gly Val Ser Gly Leu Arg
                   485                 490                 495
   Val Ala Asp Ala Ser Val Met Pro Glu Leu Val Thr Val Asn Pro Asn
               500                 505                 510
   Ile Thr Val Met Met Ile Gly Glu Arg Cys Ala Glu Leu Ile Gln Glu
           515                 520                 525
   Asn Pro Val Glu Ala Arg Gln Ser Glu Leu Val Ser Glu Ala
       530                 535                 540
<210> 11
   <211> 542
   <212> PRT
   <213> Artificial Sequence
<220>
   <223> Expressionsprodukt von Nukleinsäuresequenz KC2 M85V
<400> 11
   Met Ala Lys Gly Glu Lys Leu Asn Ile Glu Lys Lys Asp Phe Asp Tyr
   1               5                   10                  15
   Ile Val Ile Gly Gly Gly Ser Ala Gly Ala Ala Val Ala Ser Arg Leu
               20                  25                  30
   Ser Glu Asp Pro Ser Val Ser Val Ala Leu Val Glu Ala Gly Pro Asp
           35                  40                  45
   Asp Arg Gly Tyr Asp Glu Val Leu Gln Leu Asp Arg Trp Met Glu Leu
       50                  55                  60
   Leu Glu Ser Gly Leu Asp Trp Asp Tyr Pro Ile Glu Pro Gln Glu Asn
   65                  70                  75                  80
   Gly Asn Ser Phe Val Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Phe Gly Ala Thr Gly Trp Asn Ser Glu Met
           115                 120                 125
   Ala Tyr Arg Leu Tyr Lys Lys Leu Glu Thr Asn Glu Asp Ala Gly Pro
       130                 135                 140
   Asp Ala Pro His His Gly Asp Ser Gly Pro Val Lys Leu Met Asn Val
   145                 150                 155                 160
   Pro Pro Val Asp Pro Cys Gly Val Ala Ile Leu Asp Ala Ala Glu Gln
                   165                 170                 175
   Ala Gly Ile Pro Arg Ala Lys Phe Asn Asn Asn Glu Thr Val Ile Asn
               180                 185                 190
   Gly Ala Asn Phe Phe Gln Ile Asn Arg Leu Pro Asp Gly Thr Arg Ala
           195                 200                 205
   Ser Ser Ser Val Ser Tyr Ile His Pro Ile Glu Gly Arg Glu Asn Phe
       210                 215                 220
   Phe Leu Leu Thr Gly Leu Gln Ala Arg Lys Leu Asn Phe Asp Ala Asp
   225                 230                 235                 240
   Lys Arg Cys Thr Gly Val Asp Val Val Asp Gly Ala Phe Gly Arg Thr
                   245                 250                 255
   Val Thr Leu Asn Ala Ala Arg Glu Val Val Val Ser Ala Gly Ala Ile
               260                 265                 270
   Asp Ser Pro Lys Leu Leu Met Leu Ser Gly Ile Gly Pro Ala Glu His
           275                 280                 285
   Leu Lys Glu Val Gly Val Glu Val Leu Val Asp Ala Pro Gly Val Gly
       290                 295                 300
   Glu His Leu Gln Asp His Pro Glu Gly Val Ile Gln Trp Glu Ala Lys
   305 310 315 320
   Lys Pro Met Val Glu Thr Ser Thr Gln Trp Trp Glu Ile Gly Ile Phe
   325 330 335
   Ala Pro Thr Gln Glu Gly Leu Asp Arg Pro Asp Leu Met Met His Tyr
   340 345 350
   Gly Ser Val Pro Phe Asp Met His Thr Leu Arg Trp Gly Tyr Pro Thr
   355 360 365
   Ser Glu Asn Thr Phe Cys Leu Thr Pro Asn Val Thr His Ala Lys Ser
   370 375 380
   Arg Gly Thr Val Arg Leu Arg Ser Cys Asp Phe Arg Asp Lys Pro Lys
   385 390 395 400
   Val Asp Pro Arg Tyr Phe Thr Asp Pro Glu Gly His Asp Ala Arg Val
   405 410 415
   Met Thr Phe Gly Ile Arg Lys Ala Arg Glu Ile Val Ala Gln Ser Pro
   420 425 430
   Met Ala Glu Trp Ala Gly Glu Glu Gln Phe Pro Gly Lys Asp Val Gln
   435 440 445
   Thr Asp Glu Glu Ile Phe Asp Tyr Leu Arg Arg Cys His Asn Thr Val
   450 455 460
   Tyr His Pro Ala Gly Ser Val Arg Met Gly Ala Glu Asp Asp Val Met
   465 470 475 480
   Ser Pro Leu Asp Pro Gln Leu Arg Val Lys Gly Val Ser Gly Leu Arg
   485 490 495
   Val Ala Asp Ala Ser Val Met Pro Glu Leu Val Thr Val Asn Pro Asn
   500 505 510
   Ile Thr Val Met Met Ile Gly Glu Arg Cys Ala Glu Leu Ile Gln Glu
   515 520 525
   Asn Pro Val Glu Ala Arg Gln Ser Glu Leu Val Ser Glu Ala
   530 535 540
<210> 12
   <211> 542
   <212> PRT
   <213> Artificial Sequence
<220>
   <223> Expressionsprodukt von Nukleinsäuresequenz KC2 A27V
<400> 12
   Met Ala Lys Gly Glu Lys Leu Asn Ile Glu Lys Lys Asp Phe Asp Tyr
   1               5                   10                  15
   Ile Val Ile Gly Gly Gly Ser Ala Gly Ala Val Val Ala Ser Arg Leu
               20                  25                  30
   Ser Glu Asp Pro Ser Val Ser Val Ala Leu Val Glu Ala Gly Pro Asp
           35                  40                  45
   Asp Arg Gly Tyr Asp Glu Val Leu Gln Leu Asp Arg Trp Met Glu Leu
   50 55 60
   Leu Glu Ser Gly Leu Asp Trp Asp Tyr Pro Ile Glu Pro Gln Glu Asn
   65 70 75 80
   Gly Asn Ser Phe Met Arg His Ala Arg Ala Lys Val Met Gly Gly Cys
   85 90 95
   Ser Ser His Asn Ser Cys Ile Ala Phe Trp Ala Pro Arg Glu Asp Leu
   100 105 110
   Asp Glu Trp Glu Ser Lys Phe Gly Ala Thr Gly Trp Asn Ser Glu Met
   115 120 125
   Ala Tyr Arg Leu Tyr Lys Lys Leu Glu Thr Asn Glu Asp Ala Gly Pro
   130 135 140
   Asp Ala Pro His His Gly Asp Ser Gly Pro Val Lys Leu Met Asn Val
   145 150 155 160
   Pro Pro Val Asp Pro Cys Gly Val Ala Ile Leu Asp Ala Ala Glu Gln
   165 170 175
   Ala Gly Ile Pro Arg Ala Lys Phe Asn Asn Asn Glu Thr Val Ile Asn
   180 185 190
   Gly Ala Asn Phe Phe Gln Ile Asn Arg Leu Pro Asp Gly Thr Arg Ala
   195 200 205
   Ser Ser Ser Val Ser Tyr Ile His Pro Ile Glu Gly Arg Glu Asn Phe
   210 215 220
   Phe Leu Leu Thr Gly Leu Gln Ala Arg Lys Leu Asn Phe Asp Ala Asp
   225 230 235 240
   Lys Arg Cys Thr Gly Val Asp Val Val Asp Gly Ala Phe Gly Arg Thr
   245 250 255
   Val Thr Leu Asn Ala Ala Arg Glu Val Val Val Ser Ala Gly Ala Ile
   260 265 270
   Asp Ser Pro Lys Leu Leu Met Leu Ser Gly Ile Gly Pro Ala Glu His
   275 280 285
   Leu Lys Glu Val Gly Val Glu Val Leu Val Asp Ala Pro Gly Val Gly
   290 295 300
   Glu His Leu Gln Asp His Pro Glu Gly Val Ile Gln Trp Glu Ala Lys
   305 310 315 320
   Lys Pro Met Val Glu Thr Ser Thr Gln Trp Trp Glu Ile Gly Ile Phe
   325 330 335
   Ala Pro Thr Gln Glu Gly Leu Asp Arg Pro Asp Leu Met Met His Tyr
   340 345 350
   Gly Ser Val Pro Phe Asp Met His Thr Leu Arg Trp Gly Tyr Pro Thr
   355 360 365
   Ser Glu Asn Thr Phe Cys Leu Thr Pro Asn Val Thr His Ala Lys Ser
   370 375 380
   Arg Gly Thr Val Arg Leu Arg Ser Cys Asp Phe Ser Asp Lys Pro Lys
   385 390 395 400
   Val Asp Pro Arg Tyr Phe Thr Asp Pro Glu Gly His Asp Ala Arg Val
   405 410 415
   Met Thr Phe Gly Ile Arg Lys Ala Arg Glu Ile Val Ala Gln Ser Pro
   420 425 430
   Met Ala Glu Trp Ala Gly Glu Glu Gln Phe Pro Gly Lys Asp Val Gln
   435 440 445
   Thr Asp Glu Glu Ile Phe Asp Tyr Leu Arg Arg Cys His Asn Thr Val
   450 455 460
   Tyr His Pro Ala Gly Ser Val Arg Met Gly Ala Glu Asp Asp Val Met
   465 470 475 480
   Ser Pro Leu Asp Pro Gln Leu Arg Val Lys Gly Val Ser Gly Leu Arg
   485 490 495
   Val Ala Asp Ala Ser Val Met Pro Glu Leu Val Thr Val Asn Pro Asn
   500 505 510
   Ile Thr Val Met Met Ile Gly Glu Arg Cys Ala Glu Leu Ile Gln Glu
   515 520 525
   Asn Pro Val Glu Ala Arg Gln Ser Glu Leu Val Ser Glu Ala
   530 535 540
<210> 13
   <211> 542
   <212> PRT
   <213> Artificial Sequence
<220>
   <223> Expressionsprodukt von Nukleinsäuresequenz KC2 M85V2
<400> 13
   Met Ala Lys Gly Glu Lys Leu Asn Ile Glu Lys Lys Asp Phe Asp Tyr
   1               5                   10                  15
   Ile Val Ile Gly Gly Gly Ser Ala Gly Ala Ala Val Ala Ser Arg Leu
               20                  25                  30
   Ser Glu Asp Pro Ser Val Ser Val Ala Leu Val Glu Ala Gly Pro Asp
           35                  40                  45
   Asp Arg Gly Tyr Asp Glu Val Leu Gln Leu Asp Arg Trp Met Glu Leu
       50                  55                  60
   Leu Glu Ser Gly Leu Asp Trp Asp Tyr Pro Ile Glu Pro Gln Glu Asn
   65                  70                  75                  80
   Gly Asn Ser Phe Val Arg His Ala Arg Ala Lys Val Met Gly Gly Cys
                   85                  90                  95
   Ser Ser His Asn Ser Cys Ile Ala Phe Trp Ala Pro Arg Glu Asp Leu
               100                 105                 110
   Asp Glu Trp Glu Ser Lys Phe Gly Ala Thr Gly Trp Asn Ser Glu Met
           115                 120                 125
   Ala Tyr Arg Leu Tyr Lys Lys Leu Glu Thr Asn Glu Asp Ala Gly Pro
   130 135 140
   Asp Ala Pro His His Gly Asp Ser Gly Pro Val Lys Leu Met Asn Val
   145 150 155 160
   Pro Pro Val Asp Pro Cys Gly Val Ala Ile Leu Asp Ala Ala Glu Gln
   165 170 175
   Ala Gly Ile Pro Arg Ala Lys Phe Asn Asn Asn Glu Thr Val Ile Asn
   180 185 190
   Gly Ala Asn Phe Phe Gln Ile Asn Arg Leu Pro Asp Gly Thr Arg Ala
   195 200 205
   Ser Ser Ser Val Ser Tyr Ile His Pro Ile Glu Gly Arg Glu Asn Phe
   210 215 220
   Phe Leu Leu Thr Gly Leu Gln Ala Arg Lys Leu Asn Phe Asp Ala Asp
   225 230 235 240
   Lys Arg Cys Thr Gly Val Asp Val Val Asp Gly Ala Phe Gly Arg Thr
   245 250 255
   Val Thr Leu Asn Ala Ala Arg Glu Val Val Val Ser Ala Gly Ala Ile
   260 265 270
   Asp Ser Pro Lys Leu Leu Met Leu Ser Gly Ile Gly Pro Ala Glu His
   275 280 285
   Leu Lys Glu Val Gly Val Glu Val Leu Val Asp Ala Pro Gly Val Gly
   290 295 300
   Glu His Leu Gln Asp His Pro Glu Gly Val Ile Gln Trp Glu Ala Lys
   305 310 315 320
   Lys Pro Met Val Glu Thr Ser Thr Gln Trp Trp Glu Ile Gly Ile Phe
   325 330 335
   Ala Pro Thr Gln Glu Gly Leu Asp Arg Pro Asp Leu Met Met His Tyr
   340 345 350
   Gly Ser Val Pro Phe Asp Met His Thr Leu Arg Trp Gly Tyr Pro Thr
   355 360 365
   Ser Glu Asn Thr Phe Cys Leu Thr Pro Asn Val Thr His Ala Lys Ser
   370 375 380
   Arg Gly Thr Val Arg Leu Arg Ser Cys Asp Phe Arg Asp Lys Pro Lys
   385 390 395 400
   Val Asp Pro Arg Tyr Phe Thr Asp Pro Glu Gly His Asp Ala Arg Val
   405 410 415
   Met Thr Phe Gly Ile Arg Lys Ala Arg Glu Ile Val Ala Gln Ser Pro
   420 425 430
   Met Ala Glu Trp Ala Gly Glu Glu Gln Phe Pro Gly Lys Asp Val Gln
   435 440 445
   Thr Asp Glu Glu Ile Phe Asp Tyr Leu Arg Arg Cys His Asn Thr Val
   450 455 460
   Tyr His Pro Ala Gly Ser Val Arg Met Gly Ala Glu Asp Asp Val Met
   465 470 475 480
   Ser Pro Leu Asp Pro Gln Leu Arg Val Lys Gly Val Ser Gly Leu Arg
   485 490 495
   Val Ala Asp Ala Ser Val Met Pro Glu Leu Val Thr Val Asn Pro Asn
   500 505 510
   Ile Thr Val Met Met Ile Gly Glu Arg Cys Ala Glu Leu Ile Gln Glu
   515 520 525
   Asn Pro Val Glu Ala Arg Gln Ser Glu Leu Val Ser Glu Ala
   530 535 540
<210> 14
   <211> 536
   <212> PRT
   <213> Artificial Sequence
<220>
   <223> Expressionsprodukt von Nukleinsäuresequenz KC2s
<400> 14
   Met Asn Ile Glu Lys Lys Asp Phe Asp Tyr Ile Val Ile Gly Gly Gly
   1               5                   10                  15
   Ser Ala Gly Ala Ala Val Ala Ser Arg Leu Ser Glu Asp Pro Ser Val
               20                  25                  30
   Ser Val Ala Leu Val Glu Ala Gly Pro Asp Asp Arg Gly Tyr Asp Glu
           35                  40                  45
   Val Leu Gln Leu Asp Arg Trp Met Glu Leu Leu Glu Ser Gly Leu Asp
       50                  55                  60
   Trp Asp Tyr Pro Ile Glu Pro Gln Glu Asn Gly Asn Ser Phe Met Arg
   65                  70                  75                  80
   His Ala Arg Ala Lys Val Met Gly Gly Cys Ser Ser His Asn Ser Cys
                   85                  90                  95
   Ile Ala Phe Trp Ala Pro Arg Glu Asp Leu Asp Glu Trp Glu Ser Lys
               100                 105                 110
   Phe Gly Ala Thr Gly Trp Asn Ser Glu Met Ala Tyr Arg Leu Tyr Lys
           115                 120                 125
   Lys Leu Glu Thr Asn Glu Asp Ala Gly Pro Asp Ala Pro His His Gly
       130                 135                 140
   Asp Ser Gly Pro Val Lys Leu Met Asn Val Pro Pro Val Asp Pro Cys
   145                 150                 155                 160
   Gly Val Ala Ile Leu Asp Ala Ala Glu Gln Ala Gly Ile Pro Arg Ala
                   165                 170                 175
   Lys Phe Asn Asn Asn Glu Thr Val Ile Asn Gly Ala Asn Phe Phe Gln
               180                 185                 190
   Ile Asn Arg Leu Pro Asp Gly Thr Arg Ala Ser Ser Ser Val Ser Tyr
           195                 200                 205
   Ile His Pro Ile Glu Gly Arg Glu Asn Phe Phe Leu Leu Thr Gly Leu
   210 215 220
   Gln Ala Arg Lys Leu Asn Phe Asp Ala Asp Lys Arg Cys Thr Gly Val
   225 230 235 240
   Asp Val Val Asp Gly Ala Phe Gly Arg Thr Val Thr Leu Asn Ala Ala
   245 250 255
   Arg Glu Val Val Val Ser Ala Gly Ala Ile Asp Ser Pro Lys Leu Leu
   260 265 270
   Met Leu Ser Gly Ile Gly Pro Ala Glu His Leu Lys Glu Val Gly Val
   275 280 285
   Glu Val Leu Val Asp Ala Pro Gly Val Gly Glu His Leu Gln Asp His
   290 295 300
   Pro Glu Gly Val Ile Gln Trp Glu Ala Lys Lys Pro Met Val Glu Thr
   305 310 315 320
   Ser Thr Gln Trp Trp Glu Ile Gly Ile Phe Ala Pro Thr Gln Glu Gly
   325 330 335
   Leu Asp Arg Pro Asp Leu Met Met His Tyr Gly Ser Val Pro Phe Asp
   340 345 350
   Met His Thr Leu Arg Trp Gly Tyr Pro Thr Ser Glu Asn Thr Phe Cys
   355 360 365
   Leu Thr Pro Asn Val Thr His Ala Lys Ser Arg Gly Thr Val Arg Leu
   370 375 380
   Arg Ser Cys Asp Phe Ser Asp Lys Pro Lys Val Asp Pro Arg Tyr Phe
   385 390 395 400
   Thr Asp Pro Glu Gly His Asp Ala Arg Val Met Thr Phe Gly Ile Arg
   405 410 415
   Lys Ala Arg Glu Ile Val Ala Gln Ser Pro Met Ala Glu Trp Ala Gly
   420 425 430
   Glu Glu Gln Phe Pro Gly Lys Asp Val Gln Thr Asp Glu Glu Ile Phe
   435 440 445
   Asp Tyr Leu Arg Arg Cys His Asn Thr Val Tyr His Pro Ala Gly Ser
   450 455 460
   Val Arg Met Gly Ala Glu Asp Asp Val Met Ser Pro Leu Asp Pro Gln
   465 470 475 480
   Leu Arg Val Lys Gly Val Ser Gly Leu Arg Val Ala Asp Ala Ser Val
   485 490 495
   Met Pro Glu Leu Val Thr Val Asn Pro Asn Ile Thr Val Met Met Ile
   500 505 510
   Gly Glu Arg Cys Ala Glu Leu Ile Gln Glu Asn Pro Val Glu Ala Arg
   515 520 525
   Gln Ser Glu Leu Val Ser Glu Ala
   530 535
<210> 15
   <211> 536
   <212> PRT
   <213> Artificial Sequence
<220>
   <223> Expressionsprodukt von Nukleinsäuresequenz KC2s in E. coli
<400> 15
   Met Asn Ile Glu Lys Lys Asp Phe Asp Tyr Ile Val Ile Gly Gly Gly
   1               5                   10                  15
   Ser Ala Gly Ala Ala Val Ala Ser Arg Leu Ser Glu Asp Pro Ser Val
               20                  25                  30
   Ser Val Ala Leu Val Glu Ala Gly Pro Asp Asp Arg Gly Tyr Asp Glu
           35                  40                  45
   Val Leu Gln Leu Asp Arg Trp Met Glu Leu Leu Glu Ser Gly Leu Asp
       50                  55                  60
   Trp Asp Tyr Pro Ile Glu Pro Gln Glu Asn Gly Asn Ser Phe Met Arg
   65                  70                  75                  80
   His Ala Arg Ala Lys Val Met Gly Gly Cys Ser Ser His Asn Ser Cys
                   85                  90                  95
   Ile Ala Phe Trp Ala Pro Arg Glu Asp Leu Asp Glu Trp Glu Ser Lys
               100                 105                 110
   Phe Gly Ala Thr Gly Trp Asn Ser Glu Met Ala Tyr Arg Leu Tyr Lys
           115                 120                 125
   Lys Leu Glu Thr Asn Glu Asp Ala Gly Pro Asp Ala Pro His His Gly
       130                 135                 140
   Asp Ser Gly Pro Val Lys Leu Met Asn Val Pro Pro Val Asp Pro Cys
   145                 150                 155                 160
   Gly Val Ala Ile Leu Asp Ala Ala Glu Gln Ala Gly Ile Pro Arg Ala
                   165                 170                 175
   Lys Phe Asn Asn Asn Glu Thr Val Ile Asn Gly Ala Asn Phe Phe Gln
               180                 185                 190
   Ile Asn Arg Leu Pro Asp Gly Thr Arg Ala Ser Ser Ser Val Ser Tyr
           195                 200                 205
   Ile His Pro Ile Glu Gly Arg Glu Asn Phe Phe Leu Leu Thr Gly Leu
       210                 215                 220
   Gln Ala Arg Lys Leu Asn Phe Asp Ala Asp Lys Arg Cys Thr Gly Val
   225                 230                 235                 240
   Asp Val Val Asp Gly Ala Phe Gly Arg Thr Val Thr Leu Asn Ala Ala
                   245                 250                 255
   Arg Glu Val Val Val Ser Ala Gly Ala Ile Asp Ser Pro Lys Leu Leu
               260                 265                 270
   Met Leu Ser Gly Ile Gly Pro Ala Glu His Leu Lys Glu Val Gly Val
           275                 280                 285
   Glu Val Leu Val Asp Ala Pro Gly Val Gly Glu His Leu Gln Asp His
       290                 295                 300
   Pro Glu Gly Val Ile Gln Trp Glu Ala Lys Lys Pro Met Val Glu Thr
   305                 310                 315                 320
   Ser Thr Gln Trp Trp Glu Ile Gly Ile Phe Ala Pro Thr Gln Glu Gly
                   325                 330                 335
   Leu Asp Arg Pro Asp Leu Met Met His Tyr Gly Ser Val Pro Phe Asp
               340                 345                 350
   Met His Thr Leu Arg Trp Gly Tyr Pro Thr Ser Glu Asn Thr Phe Cys
           355                 360                 365
   Leu Thr Pro Asn Val Thr His Ala Lys Ser Arg Gly Thr Val Arg Leu
       370                 375                 380
   Arg Ser Cys Asp Phe Ser Asp Lys Pro Lys Val Asp Pro Arg Tyr Phe
   385                 390                 395                 400
   Thr Asp Pro Glu Gly His Asp Ala Arg Val Met Thr Phe Gly Ile Arg
                   405                 410                 415
   Lys Ala Arg Glu Ile Val Ala Gln Ser Pro Met Ala Glu Trp Ala Gly
               420                 425                 430
   Glu Glu Gln Phe Pro Gly Lys Asp Val Gln Thr Asp Glu Glu Ile Phe
           435                 440                 445
   Asp Tyr Leu Arg Arg Cys His Asn Thr Val Tyr His Pro Ala Gly Ser
       450                 455                 460
   Val Arg Met Gly Ala Glu Asp Asp Val Met Ser Pro Leu Asp Pro Gln
   465                 470                 475                 480
   Leu Arg Val Lys Gly Val Ser Gly Leu Arg Val Ala Asp Ala Ser Val
                   485                 490                 495
   Met Pro Glu Leu Val Thr Val Asn Pro Asn Ile Thr Val Met Met Ile
               500                 505                 510
   Gly Glu Arg Cys Ala Glu Leu Ile Gln Glu Asn Pro Val Glu Ala Arg
           515                 520                 525
   Gln Ser Glu Leu Val Ser Glu Ala
       530                 535
<210> 16
   <211> 547
   <212> PRT
   <213> Arthrobacter globiformis
<220>
   <223> Expressionsprodukt von Nukleinsäuresequenz Cholinoxidase aus Arthrobacter globiformis (definiert Stand der Technik)
<400> 16
   Met His Ile Asp Asn Ile Glu Asn Leu Ser Asp Arg Glu Phe Asp Tyr
   1 5 10 15
   Ile Val Val Gly Gly Gly Ser Ala Gly Ala Ala Val Ala Ala Arg Leu
   20 25 30
   Ser Glu Asp Pro Ala Val Ser Val Ala Leu Val Glu Ala Gly Pro Asp
   35 40 45
   Asp Arg Gly Val Pro Glu Val Leu Gln Leu Asp Arg Trp Met Glu Leu
   50 55 60
   Leu Glu Ser Gly Tyr Asp Trp Asp Tyr Pro Ile Glu Pro Gln Glu Asn
   65 70 75 80
   Gly Asn Ser Phe Met Arg His Ala Arg Ala Lys Val Met Gly Gly Cys
   85 90 95
   Ser Ser His Asn Ser Cys Ile Ala Phe Trp Ala Pro Arg Glu Asp Leu
   100 105 110
   Asp Glu Trp Glu Ala Lys Tyr Gly Ala Thr Gly Trp Asn Ala Glu Ala
   115 120 125
   Ala Trp Pro Leu Tyr Lys Arg Leu Glu Thr Asn Glu Asp Ala Gly Pro
   130 135 140
   Asp Ala Pro His His Gly Asp Ser Gly Pro Val His Leu Met Asn Val
   145 150 155 160
   Pro Pro Lys Asp Pro Thr Gly Val Ala Leu Leu Asp Ala Cys Glu Gln
   165 170 175
   Ala Gly Ile Pro Arg Ala Lys Phe Asn Thr Gly Thr Thr Val Val Asn
   180 185 190
   Gly Ala Asn Phe Phe Gln Ile Asn Arg Arg Ala Asp Gly Thr Arg Ser
   195 200 205
   Ser Ser Ser Val Ser Tyr Ile His Pro Ile Val Glu Gln Glu Asn Phe
   210 215 220
   Thr Leu Leu Thr Gly Leu Arg Ala Arg Gln Leu Val Phe Asp Ala Asp
   225 230 235 240
   Arg Arg Cys Thr Gly Val Asp Ile Val Asp Ser Ala Phe Gly Arg Thr
   245 250 255
   His Arg Leu Thr Ala Arg Asn Glu Val Val Leu Ser Thr Gly Ala Ile
   260 265 270
   Asp Thr Pro Lys Leu Leu Met Leu Ser Gly Ile Gly Pro Ala Ala His
           275                 280                 285
   Leu Ala Glu His Gly Ile Glu Val Leu Gly Gly Leu Pro Arg Arg Gly
       290                 295                 300
   Arg Ala Pro Ala Gly Pro Pro Gly Arg Arg Gly Ala Val Arg Gly Gln
   305                 310                 315                 320
   Ala Ala His Gly Arg Arg Val His Ala Val Val Gly Asp Arg His Leu
                   325                 330                 335
   His Pro His Arg Gly Arg Pro Gly Pro Pro Arg Pro Asp Asp Ala Leu
               340                 345                 350
   Arg Leu Arg Ala Val Arg His Glu His Pro Ala Ala Arg Leu Pro His
           355                 360                 365
   His Gly Glu Arg Ala Ser Ala Ser Pro Arg Thr Ser Arg Thr Pro Ala
       370                 375                 380
   Pro Ala Ala Leu Ser Gly Cys Ala Ala Ala Thr Ser Ala Ile Ser Pro
   385                 390                 395                 400
   Trp Ser Thr Arg Ala Thr Ser Pro Thr Gln Lys Gly His Asp Met Arg
                   405                 410                 415
   Val Met Val Ala Gly Ile Arg Lys Ala Arg Glu Ile Ala Ala Gln Pro
               420                 425                 430
   Ala Met Ala Glu Trp Thr Gly Arg Glu Leu Ser Pro Gly Val Glu Ala
           435                 440                 445
   Gln Thr Asp Glu Glu Leu Gln Asp Tyr Ile Arg Lys Thr His Asn Thr
       450                 455                 460
   Val Tyr His Pro Val Gly Thr Val Arg Met Gly Ala Val Glu Asp Glu
   465                 470                 475                 480
   Met Ser Pro Leu Asp Pro Glu Leu Arg Val Lys Gly Val Thr Gly Leu
                   485                 490                 495
   Arg Val Gly Asp Ala Ser Val Met Pro Glu His Val Thr Val Asn Pro
               500                 505                 510
   Asn Ile Thr Val Met Met Ile Gly Glu Arg Cys Ala Asp Leu Ile Arg
           515                 520                 525
   Ser Ala Arg Ala Gly Glu Thr Thr Thr Ala Asp Ala Glu Leu Ser Ala
       530                 535                 540
   Ala Leu Ala
   545
<210> 17
   <211> 1629
   <212> DNA
   <213> Artificial Sequence
<220>
   <223> Cholinoxidase aus A. nicotianiae (KC 2, DSMZ-ID 96-878) kloniert in E. coli.
<400> 17
   atggcaaagg gcgaaaaatt gaacattgaa aagaaggact tcgactacat tgtgatcggc    60
   ggcggttctg ccggcgctgc ggtggcttct cgcctgagcg aggatccatc ggtgagtgtt   120
   gcactggttg aagcaggtcc cgacgaccgc ggctacgatg aagtgctgca gctggaccgc   180
   tggatggaac tgctggaatc cggcttggac tgggactacc cgatcgaacc gcaggaaaac   240
   ggcaactcgt tcatgcgcca tgcccgtgcc aaggtcatgg gcggctgctc cagccacaac   300
   tcctgcatcg ccttctgggc gccgcgtgag gaccttgacg agtgggagtc gaagttcggc   360
   gccaccggct ggaactccga gatggcctac cgcctgtaca agaagctgga aaccaatgag   420
   gacgccggac cggatgcacc gcaccacggc gactcgggcc cggtcaagct gatgaacgtg   480
   ccgccggtgg acccctgcgg cgtggcaatc ctggacgccg ccgagcaggc aggcatcccg   540
   cgcgccaagt tcaacaacaa cgagacggtc atcaacggcg cgaacttctt ccagatcaac   600
   cgcctgccgg acggcacccg tgcctcctcc tcggtctcct acatccaccc gatcgaggga   660
   cgcgagaact tcttcctgct caccggactg caggcgcgca agctgaactt cgacgccgac   720
   aagcgctgca ccggcgtgga cgtggtggac ggggccttcg gccgcaccgt gacgctgaac   780
   gccgcccgcg aggtcgtcgt ctccgccggc gccattgact cgccgaagct gctgatgctc   840
   tccggcatcg gacctgccga acacctcaag gaggtcggcg tcgaggtgct tgtcgacgct   900
   ccaggcgtgg gcgaacacct gcaggaccac ccggaaggcg tgatccagtg ggaggctaag   960
   aagccgatgg ttgaaacctc cacccagtgg tgggaaatcg gcatcttcgc accgacccag  1020
   gaaggcctcg accgtccgga tctgatgatg cactacggtt cggtgccctt cgacatgcac  1080
   accctgcgtt ggggttaccc gaccagcgag aacaccttct gcctgacccc gaatgtcacc  1140
   catgcgaagt cgcgcggcac cgtgcgcctg cgcagctgcg acttcagcga caagccgaag  1200
   gtcgaccctc gctacttcac cgatccagag ggccacgacg cccgggtcat gaccttcggc  1260
   atccgcaagg cccgcgagat cgtcgcccag tcgccgatgg cagagtgggc tggcgaggag  1320
   cagttcccgg gcaaggacgt gcagactgac gaagagatct tcgactacct gcgccgctgc  1380
   cacaacactg tctaccaccc ggctggttcg gtgcgcatgg gcgccgagga cgatgtgatg  1440
   agcccgctgg atccgcagct gcgggtcaag ggagtctcgg gcctgcgcgt ggccgacgct  1500
   tcggtgatgc cggagctggt caccgtgaac ccgaacatca cggtgatgat gatcggtgaa  1560
   cgctgcgccg agctgattca ggaaaaccct gtggaagccc gccagagcga actggttagc  1620
   gaagcctag                                                          1629
<210> 18
   <211> 1629
   <212> DA
   <213> Artificial Sequence
<220>
   <223> KC2 M85V, Mutante von KC2 in E. coli.
<400> 18
   atggcaaagg gcgaaaaatt gaacattgaa aagaaggact tcgactacat tgtgatcggc   60
   ggcggttctg ccggcgctgc ggtggcttct cgcctgagcg aggatccatc ggtgagtgtt  120
   gcactggttg aagcaggtcc cgacgaccgc ggctacgatg aagtgctgca gctggaccgc  180
   tggatggaac tgctggaatc cggcttggac tgggactacc cgatcgaacc gcaggaaaac  240
   ggcaactcgt tcgtgcgcca tgcccgtgcc aaggtcatgg gcggctgctc cagccacaac  300
   tcctgcatcg ccttctgggc gccgcgtgag gaccttgacg agtgggagtc gaagttcggc  360
   gccaccggct ggaactccga gatggcctac cgcctgtaca agaagctgga aaccaatgag   420
   gacgccggac cggatgcacc gcaccacggc gactcgggcc cggtcaagct gatgaacgtg   480
   ccgccggtgg acccctgcgg cgtggcaatc ctggacgccg ccgagcaggc aggcatcccg   540
   cgcgccaagt tcaacaacaa cgagacggtc atcaacggcg cgaacttctt ccagatcaac   600
   cgcctgccgg acggcacccg tgcctcctcc tcggtctcct acatccaccc gatcgaggga   660
   cgcgagaact tcttcctgct caccggactg caggcgcgca agctgaactt cgacgccgac   720
   aagcgctgca ccggcgtgga cgtggtggac ggggccttcg gccgcaccgt gacgctgaac   780
   gccgcccgcg aggtcgtcgt ctccgccggc gccattgact cgccgaagct gctgatgctc   840
   tccggcatcg gacctgccga acacctcaag gaggtcggcg tcgaggtgct tgtcgacgct   900
   ccaggcgtgg gcgaacacct gcaggaccac ccggaaggcg tgatccagtg ggaggctaag   960
   aagccgatgg ttgaaacctc cacccagtgg tgggaaatcg gcatcttcgc accgacccag  1020
   gaaggcctcg accgtccgga tctgatgatg cactacggtt cggtgccctt cgacatgcac  1080
   accctgcgtt ggggttaccc gaccagcgag aacaccttct gcctgacccc gaatgtcacc  1140
   catgcgaagt cgcgcggcac cgtgcgcctg cgcagctgcg acttcagaga caagccgaag  1200
   gtcgaccctc gctacttcac cgatccagag ggccacgacg cccgggtcat gaccttcggc  1260
   atccgcaagg cccgcgagat cgtcgcccag tcgccgatgg cagagtgggc tggcgaggag  1320
   cagttcccgg gcaaggacgt gcagactgac gaagagatct tcgactacct gcgccgctgc  1380
   cacaacactg tctaccaccc ggctggttcg gtgcgcatgg gcgccgagga cgatgtgatg  1440
   agcccgctgg atccgcagct gcgggtcaag ggagtctcgg gcctgcgcgt ggccgacgct  1500
   tcggtgatgc cggagctggt caccgtgaac ccgaacatca cggtgatgat gatcggtgaa  1560
   cgctgcgccg agctgattca ggaaaaccct gtggaagccc gccagagcga actggttagc  1620
   gaagcctag                                                          1629
<210> 19
   <211> 1629
   <212> DNA
   <213> Artificial Sequence
<220>
   <223> KC2 A27V, Mutante von KC2 in E. coli.
<400> 19
   atggcaaagg gcgaaaaatt gaacattgaa aagaaggact tcgactacat tgtgatcggc   60
   ggcggttctg ccggcgctgt ggtggcttct cgcctgagcg aggatccatc ggtgagtgtt  120
   gcactggttg aagcaggtcc cgacgaccgc ggctacgatg aagtgctgca gctggaccgc  180
   tggatggaac tgctggaatc cggcttggac tgggactacc cgatcgaacc gcaggaaaac  240
   ggcaactcgt tcatgcgcca tgcccgtgcc aaggtcatgg gcggctgctc cagccacaac  300
   tcctgcatcg ccttctgggc gccgcgtgag gaccttgacg agtgggagtc gaagttcggc  360
   gccaccggct ggaactccga gatggcctac cgcctgtaca agaagctgga aaccaatgag  420
   gacgccggac cggatgcacc gcaccacggc gactcgggcc cggtcaagct gatgaacgtg  480
   ccgccggtgg acccctgcgg cgtggcaatc ctggacgccg ccgagcaggc aggcatcccg  540
   cgcgccaagt tcaacaacaa cgagacggtc atcaacggcg cgaacttctt ccagatcaac  600
   cgcctgccgg acggcacccg tgcctcctcc tcggtctcct acatccaccc gatcgaggga  660
   cgcgagaact tcttcctgct caccggactg caggcgcgca agctgaactt cgacgccgac  720
   aagcgctgca ccggcgtgga cgtggtggac ggggccttcg gccgcaccgt gacgctgaac  780
   gccgcccgcg aggtcgtcgt ctccgccggc gccattgact cgccgaagct gctgatgctc  840
   tccggcatcg gacctgccga acacctcaag gaggtcggcg tcgaggtgct tgtcgacgct  900
   ccaggcgtgg gcgaacacct gcaggaccac ccggaaggcg tgatccagtg ggaggctaag  960
   aagccgatgg ttgaaacctc cacccagtgg tgggaaatcg gcatcttcgc accgacccag 1020
   gaaggcctcg accgtccgga tctgatgatg cactacggtt cggtgccctt cgacatgcac 1080
   accctgcgtt ggggttaccc gaccagcgag aacaccttct gcctgacccc gaatgtcacc 1140
   catgcgaagt cgcgcggcac cgtgcgcctg cgcagctgcg acttcagcga caagccgaag 1200
   gtcgaccctc gctacttcac cgatccagag ggccacgacg cccgggtcat gactttcggc 1260
   atccgcaagg cccgcgagat cgtcgcccag tcgccgatgg cagagtgggc tggcgaggag 1320
   cagttcccgg gcaaggacgt gcagactgac gaagagatct tcgactactt gcgccgctgc 1380
   cacaacactg tctaccaccc ggctggttcg gtgcgcatgg gcgccgagga cgatgtgatg 1440
   agcccgctgg atccgcagct gcgggtcaag ggagtctcgg gcctgcgcgt ggccgacgct 1500
   tcggtgatgc cggagctggt caccgtgaac ccgaacatca cggtgatgat gatcggtgaa 1560
   cgctgcgccg agctgattca ggaaaaccct gtggaagccc gccagagcga actggttagc 1620
   gaagcctag                                                         1629
<210> 20
   <211> 1629
   <212> DNA
   <213> Artificial Sequence
<220>
   <223> KC2 M85V2, Mutante von KC2 M85V
<400> 20
   atggcaaagg gcgaaaaatt gaacattgaa aagaaggact tcgactacat tgtgatcggc   60
   ggcggttctg ccggcgctgc ggtggcttct cgcctgagcg aggatccatc ggtgagtgtt  120
   gcactggttg aagcaggtcc cgacgaccgc ggctacgatg aagtgctgca gctggaccgc  180
   tggatggaac tgctggaatc cggcttggac tgggactacc cgatcgaacc gcaggaaaac  240
   ggcaactcgt tcgtgcgcca tgcccgtgcc aaggtcatgg gcggctgctc cagccacaac  300
   tcctgcatcg ccttctgggc gccgcgtgag gaccttgacg agtgggagtc gaagttcggc  360
   gccaccggct ggaactccga gatggcctac cgcctgtaca agaagctgga aaccaatgag  420
   gacgccggac cggatgcacc gcaccacggc gactcgggcc cggtcaagct gatgaacgtg  480
   ccgccggtgg acccctgcgg cgtggcaatc ctggacgccg ccgagcaggc aggcatcccg  540
   cgcgccaagt tcaacaacaa cgagacggtc atcaacggcg cgaacttctt ccagatcaac  600
   cgcctgccgg atggcacccg tgcctcctcc tcggtctcct acatccaccc gatcgaggga  660
   cgcgagaact tcttcctgct caccggactg caggcgcgca agctgaactt cgacgccgac  720
   aagcgctgca ccggcgtgga cgtggtggac ggggccttcg gccgcaccgt gacgctgaac  780
   gccgcccgcg aggtcgtcgt ctccgccggc gccattgact cgccgaagct gctgatgctc  840
   tccggcatcg gacctgccga acacctcaag gaggtcggcg tcgaggtgct tgtcgacgct  900
   ccaggcgtgg gcgaacacct gcaggaccac ccggaaggcg tgatccagtg ggaggctaag  960
   aagccgatgg ttgaaacctc cacccagtgg tgggaaatcg gcatcttcgc accgacccag 1020
   gaaggcctcg accgtccgga tctgatgatg cactacggtt cggtgccctt cgacatgcac 1080
   accctgcgtt ggggttaccc gaccagcgag aacaccttct gcctgacccc gaatgtcacc 1140
   catgcgaagt cgcgcggcac cgtgcgcctg cgcagctgcg acttcagaga caagccgaag 1200
   gtcgaccctc gctacttcac cgatccagag ggccacgacg cccgggtcat gaccttcggc 1260
   atccgcaagg cccgcgagat cgtcgcccag tcgccgatgg cagagtgggc tggcgaggag 1320
   cagttcccgg gcaaggacgt gcagactgac gaagagatct tcgactacct gcgccgctgc 1380
   cacaacactg tctaccaccc ggctggttcg gtgcgcatgg gcgccgagga cgatgtgatg 1440
   agcccgctgg atccgcagct gcgggtcaag ggagtctcgg gcctgcgcgt ggccgacgct 1500
   tcggtgatgc cggagctggt caccgtgaac ccgaacatca cggtgatgat gatcggtgaa 1560
   cgctgcgccg agctgattca ggaaaaccct gtggaagccc gccagagcga actggttagc 1620
   gaagcctag                                                         1629
<210> 21
   <211> 1611
   <212> DNA
   <213> Artificial Sequence
<220>
   <223> KC2s (Urform aus KC2 abgeleitet)
<400> 21
   ttgaacattg aaaagaagga cttcgactac attgtgatcg gcggcggttc tgccggcgct   60
   gcggtggctt ctcgcctgag cgaggatcca tcggtgagtg ttgcactggt tgaagcaggt  120
   cccgacgacc gcggctacga tgaagtgctg cagctggacc gctggatgga actgctggaa  180
   tccggcttgg actgggacta cccgatcgaa ccgcaggaaa acggcaactc gttcatgcgc  240
   catgcccgtg ccaaggtcat gggcggctgc tccagccaca actcctgcat cgccttctgg  300
   gcgccgcgtg aggaccttga cgagtgggag tcgaagttcg gcgccaccgg ctggaactcc  360
   gagatggcct accgcctgta caagaagctg gaaaccaatg aggacgccgg accggatgca  420
   ccgcaccacg gcgactcggg cccggtcaag ctgatgaacg tgccgccggt ggacccctgc  480
   ggcgtggcaa tcctggacgc cgccgagcag gcaggcatcc cgcgcgccaa gttcaacaac  540
   aacgagacgg tcatcaacgg cgcgaacttc ttccagatca accgcctgcc ggacggcacc  600
   cgtgcctcct cctcggtctc ctacatccac ccgatcgagg gacgcgagaa cttcttcctg  660
   ctcaccggac tgcaggcgcg caagctgaac ttcgacgccg acaagcgctg caccggcgtg  720
   gacgtggtgg acggggcctt cggccgcacc gtgacgctga acgccgcccg cgaggtcgtc  780
   gtctccgccg gcgccattga ctcgccgaag ctgctgatgc tctccggcat cggacctgcc  840
   gaacacctca aggaggtcgg cgtcgaggtg cttgtcgacg ctccaggcgt gggcgaacac  900
   ctgcaggacc acccggaagg cgtgatccag tgggaggcta agaagccgat ggttgaaacc  960
   tccacccagt ggtgggaaat cggcatcttc gcaccgaccc aggaaggcct cgaccgtccg 1020
   gatctgatga tgcactacgg ttcggtgccc ttcgacatgc acaccctgcg ttggggttac 1080
   ccgaccagcg agaacacctt ctgcctgacc ccgaatgtca cccatgcgaa gtcgcgcggc   1140
   accgtgcgcc tgcgcagctg cgacttcagc gacaagccga aggtcgaccc tcgctacttc   1200
   accgatccag agggccacga cgcccgggtc atgaccttcg gcatccgcaa ggcccgcgag   1260
   atcgtcgccc agtcgccgat ggcagagtgg gctggcgagg agcagttccc gggcaaggac   1320
   gtgcagactg acgaagagat cttcgactac ctgcgccgct gccacaacac tgtctaccac   1380
   ccggctggtt cggtgcgcat gggcgccgag gacgatgtga tgagcccgct ggatccgcag   1440
   ctgcgggtca agggagtctc gggcctgcgc gtggccgacg cttcggtgat gccggagctg   1500
   gtcaccgtga acccgaacat cacggtgatg atgatcggtg aacgctgcgc cgagctgatt   1560
   caggaaaacc ctgtggaagc ccgccagagc gaactggtta gcgaagccta g            1611
<210> 22
   <211> 1611
   <212> DNA
   <213> Artificial Sequence
<220>
   <223> KC2s (Kloniert in E. coli)
<400> 22
   atgaacattg aaaagaagga cttcgactac attgtgatcg gcggcggttc tgccggcgct   60
   gcggtggctt ctcgcctgag cgaggatcca tcggtgagtg ttgcactggt tgaagcaggt   120
   cccgacgacc gcggctacga tgaagtgctg cagctggacc gctggatgga actgctggaa   180
   tccggcttgg actgggacta cccgatcgaa ccgcaggaaa acggcaactc gttcatgcgc   240
   catgcccgtg ccaaggtcat gggcggctgc tccagccaca actcctgcat cgccttctgg   300
   gcgccgcgtg aggaccttga cgagtgggag tcgaagttcg gcgccaccgg ctggaactcc   360
   gagatggcct accgcctgta caagaagctg gaaaccaatg aggacgccgg accggatgca   420
   ccgcaccacg gcgactcggg cccggtcaag ctgatgaacg tgccgccggt ggacccctgc   480
   ggcgtggcaa tcctggacgc cgccgagcag gcaggcatcc cgcgcgccaa gttcaacaac   540
   aacgagacgg tcatcaacgg cgcgaacttc ttccagatca accgcctgcc ggacggcacc   600
   cgtgcctcct cctcggtctc ctacatccac ccgatcgagg gacgcgagaa cttcttcctg   660
   ctcaccggac tgcaggcgcg caagctgaac ttcgacgccg acaagcgctg caccggcgtg   720
   gacgtggtgg acggggcctt cggccgcacc gtgacgctga acgccgcccg cgaggtcgtc   780
   gtctccgccg gcgccattga ctcgccgaag ctgctgatgc tctccggcat cggacctgcc   840
   gaacacctca aggaggtcgg cgtcgaggtg cttgtcgacg ctccaggcgt gggcgaacac   900
   ctgcaggacc acccggaagg cgtgatccag tgggaggcta agaagccgat ggttgaaacc   960
   tccacccagt ggtgggaaat cggcatcttc gcaccgaccc aggaaggcct cgaccgtccg   1020
   gatctgatga tgcactacgg ttcggtgccc ttcgacatgc acaccctgcg ttggggttac   1080
   ccgaccagcg agaacacctt ctgcctgacc ccgaatgtca cccatgcgaa gtcgcgcggc   1140
   accgtgcgcc tgcgcagctg cgacttcagc gacaagccga aggtcgaccc tcgctacttc   1200
   accgatccag agggccacga cgcccgggtc atgaccttcg gcatccgcaa ggcccgcgag   1260
   atcgtcgccc agtcgccgat ggcagagtgg gctggcgagg agcagttccc gggcaaggac   1320
   gtgcagactg acgaagagat cttcgactac ctgcgccgct gccacaacac tgtctaccac   1380
   ccggctggtt cggtgcgcat gggcgccgag gacgatgtga tgagcccgct ggatccgcag   1440
   ctgcgggtca agggagtctc gggcctgcgc gtggccgacg cttcggtgat gccggagctg   1500
   gtcaccgtga acccgaacat cacggtgatg atgatcggtg aacgctgcgc cgagctgatt   1560
   caggaaaacc ctgtggaagc ccgccagagc gaactggtta gcgaagccta g            1611
<210> 23
   <211> 40
   <212> DNA
   <213> Artificial Sequence
<220>
   <223> KC2spETNF - Primer zur Erzeugung von KC2s
<400> 23
   ttccatatga acattgaaaa gaaggacttc gactacattg 40

## Beschreibung

Die vorliegende Erfindung betrifft neue Cholinoxidasen, Verfahren zu Ihrer Gewinnung, Körperpflegemittel, Haarpflegemittel, Haarwaschmittel, Mund-, Zahn-und Zahnprothesenpflegemittel, Kosmetika, Waschmittel, Reinigungsmittei. Nachspülmittel, Handwaschmittel, Handgeschirrspülmittel und Maschinengeschirrspülmittel, enthaltende neue Cholinoxidasen sowie Verwendungen der neuen Cholinoxidasen

Cholinoxidasen sind aus dem Stand der Technik bekannt (Ikuta, S., Imamura, S., Misaki, H., and Horiuti, Y. 1977. Purification and characterization of choline oxidase from Arthrobacter globiformis. J Biochem (Tokyo) 82: 1741-1749., Deshnium, P., Los, D.A., Hayashi, H., Mustardy, L., and Murata, N. 1995. Transformation of Synechococcus with a gene for choline oxidase enhances tolerance to salt stress. Plant Mol Biol 29: 897-907.). Ferner offenbart die internationale Patentanmeldung WO99/37786 eine bakterielle Cholinoxidase und deren Verwendung in transgenen Pflanzen. Bekannt ist auch, daß Oxidase (Alkoholoxidasen, Aminosäureoxidasen) zusammen mit ihren Substraten für die Wasserstoffperoxidgenerierung zur Bleiche und Farbübertragungsinhiblerung in Waschmitteln oder zur enzymatischen Haarfärbung und Bleiche in kosmetischen Mitteln eingesetzt werden können.

In der WO 97/21796 wird beschrieben, daß Oxidasen aus ihren entsprechenden Substraten unter technischen Bedingungen (z.B. einer Waschmittelmatrix) mit Hilfe von, Luftsauerstoff Wasserstoffperoxid freisetzen und damit zur Bleiche eingesetzt werden können. Die Bildung von Wasserstoffperoxid erfolgt kontinuierlich wobei die Effizienz der Produktbildung durch die Temperatur- und pH-Stabilität, sowie die Toleranz gegenüber Substrat und Produkt bestimmt wird.

Für die herkömmliche chemische Bleiche werden anorganische, alkalische Wasserstoffperoxidlieferanten wie Percarbonat oder Perborat In Kombination mit Bleichboostern (TAED) eingesetzt. Die Bleichkomponente Wasserstoffperoxid entsteht durch spontanen Zerfall der Addi4onsverbindung und führt damit schnell aber kurzfristig zu hohen Konzentrationen. Eine schonende, Bleiche ist nicht möglich.

Für den Einsatz in wässrigen Flüssigformulieningen steht kein optimales Bleichsystem zur Verfügung.

Für die Haarfärbung wird herkömmlich eine Bleiche zur Nivellierung von Grautönen vor der Färbung mit Wasserstoffperoxid und Ammoniaklösung durchgeführt. Die kurzfristig hohen Wasserstoffperoxidkonzentrationen in Kombination mit dem alkalischen pH führen zu einer deutlichen Haarschädigung. Eine Vorbehandlung ohne geruchsbelästigen Ammoniak mit kontinuirlicher Wasserstoffperoxidfreisetzung bei neutralem oder schwach basischem pH steht nicht zur Verfügung.

Überraschenderweise wurde gefunden, daß sich aus den Bakterien Arthrobacter nicotianiae und Arthrobacter aurescens Cholinoxidasen isolieren lassen, die unter weitgehender Vermeidung der dem einschlägigen Stand der Technik anhaftenden Nachteile in Bleichsystemen eingesetzt werden können.

Gegenstand der vorliegenden Erfindung ist daher eine Cholinoxidase, wie in den Ansprüchen 1 und 2 definiert.

Die erfindungsgemäßen Cholinoxidasen basieren auf neuen, durch die Anmelderin erstmals Isolierten alkalischen Oxidasen aus Arthrobacter nicotianiae (Seq. 1).

Die erfindungsgemäßen Cholinoxidasen sind befähigt, aus Cholin und Cholinderivaten mit Hilfe von Luftsauerstoff unter Bildung von Betainaldehyd und Betain kontinuierlich Wasserstoffperoxid freizusetzen.

Die erfindungsgemäßen Cholinoxidasen weisen vorteilhafterweise eine hohe spezifische Wasserstoffildungsrate auf.

Das pH-Profil der erfindungsgemäßen Enzyme ist kompatibel mit dem erforderlichen pH bei technischem Einsatz sowie mit typischen Produkten wie Wasch- und Reinigungsmitteln und Haarfärbungen.

Durch den Einsatz des Substrates Cholin und seiner Derivate sowie das sich bildende Nebenprodukt Betain als potentielle Wirksubstanz zur Avivage ergibt sich ein beträchtlicher Sekundämutzen.

Als Substrate kommen beispielsweise Cholin und Derivate von N-substituiertem Aminoethanol in Frage, mit den Strukturformeln 1 oder 2:

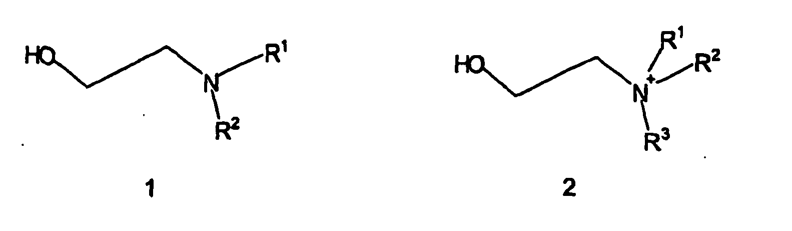

Für Strukturformel 1 gilt:
R¹ = H, R² = 2-Hydroxyethyl
R¹ = Methyl, R² = Methyl
R¹ = 2-Hydroxyethyl, R² = 2-Hydroxyethyl
Diese Verbindungen sind als Substrate für die Cholinoxidase aus A**.** globiformis literaturbekannt.

Für Strukturformel 2 gilt
R¹ = R² = R³ = Methyl

Ein weiteres erfindungsgemäß geeignetes Substrat ist Betainaldehyd (OHC-CH₂)N⁺(CH₃)₃

Von erheblichem Interesse ist auch die Zusatzwirkung des sich bildenden Betains für die Haarpflege.

Im folgenden bedeutet ein Ausdruck der Form "mindestens X %" "X % bis 100 % (einschließlich der Eckwerte X und 100 und aller ganzzahligen und nicht ganzzahligen Prozentwerte dazwischen)".

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet

Unter einem Enzym ist im Sinne der vorliegenden Anmeldung ein Protein zu verstehen, das eine bestimmte biokatalytische Funktion ausübt.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-termlnalen Aminosäuren ausübt.

Für technische Anwendungen sind aufgrund ihrer enzymatischen Aktivität die maturen Peptide, das heißt die nach ihrer Herstellung prozessierten Enzyme gegenüber den Pr-äproteinen bevorzugt.

Pro-Proteine sind inaktive Vorstufen von Proteinen. Deren Vorläufer mit Signalsequenz werden als Prä-Pro-Proteine bezeichnet.

Unter Nukleinsäuren sind im Sinne der vorliegenden Anmeldung die natürlicherweise aus Nukteotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastem zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen. Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informaüonseinheit wird auch im Sinne der vorliegenden Anmeldung als Gen bezeichnet.

Die vorliegende Erfindung umfaßt die Herstellung rekombinanter Proteine. Hierunter sind erfindungsgemäß alle gentechnische oder mikrobiologischen Verfahren zu verstehen, die darauf beruhen, daß die Gene für die interessierenden Proteine in einen für die Produktion geeigneten Wirtsorganismus eingebracht und von diesem transkribiert und translatiert werden. Geeigneterweise erfolgt die Einschleusung der betreffenden Gene über Vektoren, insbesondere Expressionsvektoren; aber auch über solche, die bewirken, daß das interessierende Gen im Wirtsorganismus in ein bereits vorhandenes genetisches Element wie das Chromosom oder andere Vektoren eingefügt werden kann. Die funktionelle Einheit aus Gen und Promotor und eventuellen weiteren gegentischen Elementen wird erfindungsgemäß als Expressionskassette bezeichnet Sie muß dafür jedoch nicht notwendigerweise auch als physische Einheit vorliegen.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press.bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert oder rekombiniert werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, inserdons-Substitutionsmutationen oder Fusionen zu deletions-, insertionssubstitutionsmutierten oder Fusionsgenen und auf Proteinebene zu entsprechenden Deletions-, Insertions- oder Substitutionsvarianten, beziehungsweise Fusionsproteinen.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Den Fragmenten entsprechen auf Nukleinsäure-Ebene die Teilsequenzen.

Unter chimären oder hybriden Proteinen sind im Sinne der vorliegenden Anmeldung solche Proteine zu verstehen, die von Nukleinsäureketten kodiert werden, die natürlicherweise von verschiedenen oder aus demselben Organismus stammen. Dieses Vorgehen wird auch Rekombinationsmutagenese genannt. Der Sinn einer solchen Rekombination kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können..

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der insertion angesehen werden. Dasselbe gilt für eine von der ursprünglichen Aminosäureabfolge abweichende Neugruppierung verschiedener Molekülteile. Sie kann sowohl als Deletionsvarlante, als Insertionsvariante, als auch als Shuffling-Variante des ursprünglichen Proteins angesehen werden.

Unter Derivaten werden im Sinne der vorliegende Anmeldung solche Proteine verstanden, deren reine Aminosäurekette chemisch modifiziert worden ist. Solche Derivatisierungen können beispielsweise biologisch im Zusammenhang mit der Proteinblosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt Dies kann beispielsweise für den Zeitraum der Lagerung sinnvoll sein. Ebenso ist unter Derivatisierung die kovalente Bindung an einen makromolekularen Träger zu verstehen.

Proteine können auch über die Reaktion mit einem Antiserum oder einem bestimmten Antikörper zu Gruppen immunologisch verwandter Proteine zusammengefaßt werden. Die Angehörigen einer Gruppe zeichnen sich dadurch aus, daß sie dieselbe, von einem Antikörper erkannte antigene Determinante aufweisen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Kdonierungsvektoren, und andererseits denen, die die Funktion erfüllen, das interessierende Gen in der Wirtszelle zu realisieren, das heißt, die Expression des betreffenden Proteins zu ermöglichen. Diese Vektoren werden als Expressionsvektoren bezeichnet.

Durch Vergleich mit bekannten Enzymen, die beispielsweise in allgemein zugänglichen Datenbanken hinterlegt sind, läßt sich aus der Aminosäure- oder Nukleotid-Sequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Solch ein Vergleich geschieht dadurch, daß ähnliche Abfolgen in den Nukleotid-oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastem zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben.

Eine Zusammenstellung aller in den verglichenen Sequenzen übereinstimmenden Positionen wird als Konsensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von Prozent Ähnlichkeit die Rede. Solche Aussagen können Ober ganze Proteine oder Gene oder nur über einzelne Bereiche getroffen werden.

Die Erstellung eines Alignments ist der erste Schritt zur Definition eines Sequenzraums. Dieser hypothetische Raum umfaßt sämtliche durch Permutation in Einzelpositionen abzuleitenden Sequenzen, die sich unter Berücksichtigung aller in den betreffenden Einzelpositionen des Alignments auftretenden Variationen ergeben. Jedes hypothetisch möglichen Proteinmolekül bildet einen Punkt in diesem Sequenzraum. Beispielsweise begründen zwei Aminosäuresequenzen, die bei weitgehender Identität an lediglich zwei verschiedenen Stellen jeweils zwei verschiedene Aminosäuren aufweisen, somit einen Sequenzraum von vier verschiedenen Aminosäuresequenzen. Ein sehr großer Sequenzraum wird erhalten, wenn zu einzelnen Sequenzen eines Raums jeweils weitere homologe Sequenzen gefunden werden. Über solche, jeweils paarweise bestehenden hohen Homologien können auch sehr niedrig homologe Sequenzen als einem Sequenzraum zugehörig erkannt werden.

Homologe Bereiche von verschiedenen Proteinen sind durch Übereinstimmungen in der Aminosäuresequenz definiert. Diese können auch durch identische Funktion gekennzeichnet sein. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübte Funktion zu verstehen, wie beispielsweise die Ausbildung einzelner Wasserstoffbrückenbindungen zur Komplexierung eines Substrats oder Übergangskomplexes.

Im Rahmen der vorliegenden Erfindung wird die Nukleinsäure geeigneterweise in einen Vektor kloniert. Die molekularbiologische Dimension der Erfindung besteht somit in Vektoren mit den Genen für die entsprechenden Proteine. Dazu können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Die Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Proteine. Sie stellen insofern Ausführungsformen der vorliegenden Erfindung dar, als die Sequenzen der enthaltenen erfindungsgemäßen Nukleinsäurebereiche jeweils innerhalb der oben näher bezeichneten Homologiebereiche liegen.

Bevorzugte Ausführungsformen der vorliegenden Erfindung sind Klonierungsvektoren. Diese eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfählge Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Expressionsvektoren sind chemisch den Klonierungsvektoren ähnlich, unterscheiden sich aber in jenen Teilsequenzen, die sie dazu befähigen, in den für die Produktion von Proteinen optimierten Wirtsorganismen zu replizieren und dort das enthaltene Gen zur Expression zu bringen. Bevorzugte Ausführungsformen sind Expressionsvektoren, die selbst die zur Expression notwendigen genetischen Elemente tragen. Die Expression wird beispielsweise von Promotoren beeinflußt, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, daß das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, daß natürliche, mit der Translation in einem Zusammenhang stehende Modifikationsreaktionen an dem entstehenden Protein genauso durchgeführt werden, wie sie auch natürlicherweise ablaufen würden.

Ausführungsformen der vorliegenden Erfindung können auch zellfreie Expressionssysteme sein, bei denen die Proteinbiosynthese in vitro nachvollzogen wird. Derartige Expressionssysteme sind im Stand der Technik ebenfalls etabliert.

Die In-vivo-Synthese eines erfindungsgemäßen Enzyms, also die durch lebende Zellen, erfordert den Transfer des zugehörigen Gens in eine Wirtszelle, deren sogenannte Transformation. Als Wirtszellen eignen sich prinzipiell alle Organismen, das heißt Prokaryonten oder Eukaryonten. Bevorzugt sind solche Wirtszellen, die sich genetisch gut handhaben lassen, was beispielsweise die Transformation mit dem Expressionsvektor und dessen stabile Etablierung angeht, beispielsweise einzeilige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Proteine.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden. Bei gram-negativen Bakterien, wie beispielsweise Escherichia coli (E. coli), werden eine Vielzahl von Proteinen in den periplasmatischen Raum sekretiert, also in das Kompartiment zwischen den beiden die Zellen einschließenden Membranen. Dies kann für spezielle Anwendungen vorteilhaft sein. Grampositive Bakterien, wie beispielsweise Bacilli oder Actinomyceten oder andere Vertreter der Actinomycetales, besitzen demgegenüber keine äußere Membran, so daß sekretierte Proteine sogleich in das die Zellen umgebende Nährmedium abgegeben werden, aus welchem sich nach einer anderen bevorzugten Ausführungsform die exprimierten erfindungsgemäßen Proteine direkt aufreinigen lassen.

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen enzymatische Funktion zu beeinflussen. Dabei kann es sich beispielsweise um andere Proteine oder Enzyme, um Inhibitoren oder um solche Elemente handeln, die die Wechselwirkung mit verschiedenen Substraten beinflussen.

Aufgrund der weitreichenden Erfahrungen, die man beispielsweise hinsichtlich der molekularbiologischen Methoden und der Kultivierbarkeit mit coliformen Bakterien hat, stellen diese bevorzugte Ausführungsformen der vorliegenden Erfindung dar. Besonders bevorzugt sind solche der Gattungen Escherichia coli, Insbesondere nichtpathogene, für die biotechnologische Produktion geeignete Stämme.

Repräsentative Vertreter dieser Gattungen sind die K12-Derivate und die B-Stämme von Escherichia coli. Stämme, die sich nach an sich bekannten genetischen und/oder mikrobiologischen Methoden von diesen ableiten lassen, und somit als deren Derivate angesehen werden können, besitzen für genetische und mikrobiologische Arbeiten die größte Bedeutung und werden vorzugsweise zur Entwicklung erfindungsgemäßer Verfahren eingesetzt. Solche Derivate können beispielsweise über Deletions- oder Insertionsmutagenese hinsichtlich ihrer Anforderungen an die Kulturbedingungen verändert sein, andere oder zusätzliche Selektionsmarker aufweisen oder andere oder zusätzliche Proteine exprimieren. Es kann sich insbesondere um solche Derivate handeln, die zusätzlich zu dem erfindungsgemäß hergestellten Protein weitere wirtschaftlich interessante Proteine exprimieren.

Auch solche Mikroorganismen sind bevorzugt, die dadurch gekennzeichnet sind, daß sie nach Transformation mit einem der oben beschriebenen Vektoren erhalten worden sind. Dabei kann es sich beispielsweise um Klonierungsvektoren handeln, die zur Lagerung und/oder Modifikation in einen beliebigen Bakterienstamm eingebracht worden sind. Solche Schritte sind in der Lagerung und in der Weiterentwicklung betreffender genetischer Elemente allgemein verbreitet. Da aus diesen Mikroorganismen die betreffenden genetischen Elemente in zur Expression geeignete gram-negative Bakterien unmittelbar übertragen werden können, handelt es sich auch bei den vorangegenagenen Transformationsprodukten um Verwirklichungen des betreffenden Erfindungsgegenstands.

Auch eukaryontische Zellen können sich zur Produktion erfindungsgemäßer Proteine eignen. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride.

Die Wirtszellen des erfindungsgemäßen Verfahrens werden in an sich bekannter Weise kultiviert und fermentiert, beispielsweise in diskontinuierlichen oder kontinuierlichen Systemen. Im ersten Fall wird ein geeignetes Nährmedium mit den Organismen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen tellweise absterben aber auch nachwachsen und gleichzeitig Produkt aus dem Medium entnommen werden kann.

Fermentationsverfahren sind an sich aus dem Stand der Technik wohlbekannt und stellen den eigentlichen großtechnischen Produktionsschritt dar, gefolgt von einer geeigneten Aufreinigungsmethode.
Alle Fermentationsverfahren, die auf einem der oben ausgeführten Verfahren zur Herstellung der rekombinanten Proteine beruhen, stellen entsprechend bevorzugte Ausführungsformen dieses Erfindungsgegenstandes dar.

Hierbei müssen die für die eingesetzten Herstellungsverfahren, für die Wirtszellen und/oder die herzustellenden Proteine jeweils optimalen Bedingungen anhand der zuvor optimierten Kulturbedingungen der betreffenden Stämme nach dem Wissen des Fachmanns, beispielsweise hinsichlich Fermentationsvolumen, Medienzusammensetzung, Sauerstoffversorgung oder Rührergeschwindigkeit experimentell ermittelt werden.

Fermentationsverfahren, die dadurch gekennzeichnet sind, daß die Fermentation über eine Zulaufstrategie durchgeführt wird, kommen ebenfalls in Betracht. Hierbei werden, die Medienbestandteile, die durch die fortlaufende Kultivierung verbraucht werden, zugefüttert; man spricht auch von einer Zufütterungsstrategie. Hierdurch können beträchtliche Steigerungen sowohl in der Zelldichte, als auch in der Biotrockenmasse und/oder vor allem der Aktivität des interessierenden Proteins erreicht werden.

Analog dazu kann die Fermentation auch so gestaltet werden, daß unerwünschte Stoffwechselprodukte herausgefiltert oder durch Zugabe von Puffer oder jeweils passende Gegenionen neutralisiert werden.

Das hergestellte Protein kann nachträglich aus dem Fermentationsmedium geerntet werden. Dieses Fermentationsverfahren ist gegenüber der Produktaufbereitung aus der Trockenmasse bevorzugt, erfodert jedoch die Zurverfügungstellung geeigneter Sekretionsmarker und Transportsysteme.

Ohne Sekretion ist u.U. die Aufreinigung des Proteins aus der Zellmasse erforderlich, auch dazu sind verschiedene Verfahrer bekannt, wie Fällung z.B durch Ammoniumsulfat oder Ethanol, oder die chromatographische Reinigung, wenn erforderlich bis zur Homogenität Die Mehrzahl der beschriebenen technischen Verfahren dürfte jedoch mit einer angereicherten, stabilisierten Präparation auskommen.

Alle bereits oben ausgeführten Elemente können zu Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar. Das optimale Verfahren muß für jeden konkreten Einzelfall experimentell ermittelt werden.

Durch Expression oder Klonierung können die erfindungsgemäßen Cholinoxidasen in der für den technischen Einsatz erforderlichen Menge zur Verfügung gestellt werden.

Ein weiterer Gegenstand der vorliegenden Erfindung ist daher eine Nukleinsäure, die für eine erfindungsgemäße Cholinoxidase kodiert, deren Aminosäuresequenz einen Teil enthält, der mit einer der in Seq. 1 oder 10-15 angegebenen Aminosäuresequenzen zu mindestens 90 %, besonders bevorzugt mindestens 95 % und ganz besonders bevorzugt zu 100 % identisch ist.

Ein weiterer Gegenstand der vorliegenden Erfindung ist eine für eine erfindungsgemäße Cholinoxidase kodierende Nukleinsäure, deren Nukleotidsequenz mit der in Seq. 4 angegebenen Nukleotidsequenz zu mindestens 85 %, insbesondere mindestens 90 %, besonders bevorzugt mindestens 95 % und ganz besonders bevorzugt zu 100 % übereinstimmt.

Die erfindungsgemäßen Cholinoxidasen weisen ein pH-Optimum vorzugsweise im fast neutralen bis schwach alkalischen Bereich von etwa pH 6 bis pH 10. besonders bevorzugt pH 7 bis pH 9 auf. Die Aktivität solcher Enzyme wird üblicherweise in U ausgedrückt, wobei die Einheit ("Unit") derjenigen Enzymmenge entspricht, die 1 µmol an Wasserstoffperoxid (H₂O₂) bei einem festgelegte pH und einer festgelegten Temperatur in 1 Minute generiert. Für die erfindungsgemäßen Cholinoxidasen bezieht sich dies auf einen pH von 9,5 und eine Temperatur von 30°C in dem unter Beispiel 6 angegebenen Verfahren.

Das Temperatur-Optimum der erfindungsgemäßen Cholinoxidasen liegt etwa im Bereich von 20 bis 60°C, insbesondere bei etwa 30°C.

Eine erfindungsgemäße Cholinoxidase wird vorzugsweise in solchen Mengen eingesetzt, daß das gesamte Mittel eine Oxidase-Aktivität von 3 U/g bis 20 000 U/g, bevorzugt von 5 U/g bis 20 000 U/g, insbesondere von 10 U/g bis 15 000 U/g, besonders bevorzugt von 10 U/g bis 1000 U/g ganz besonders bevorzugt von 10 bis 200 U/g aufweist. Mittel mit Oxidase-Aktivitäten in den genannten Bereichen weisen eine für übliche europäische maschinelle Waschverfahren ausreichend rasche Wasserstoffperoxidfreisetzung auf, wohingegen eine Steigerung der enthaltenen Oxidasemenge auf höhere Aktivitäten in der Regel keine entsprechend hohe Steigerung der Bleichleistung ergibt.

Die Menge des im erfindungsgemäßen Waschmittel enthaltenen Substrats für die Oxidase richtet sich nach der zum Erzielen des gewünschten Bleichergebnisses erforderlichen Wasserstoffperoxidmenge. Hier kann als Anhaltspunkt dienen, daß bei Enzym-Substrat Systemen, die pro Mol umgesetztem Substrat bis zu zwei Mol Wasserstoffperoxid freisetzen. Die Anwesenheit von etwa 0,05 Gew.-% bis 1 Gew.-% des Substrats in der Wasch-, Bleich- oder Reinigungsflotte in der Regel zur Erzielung eines guten Bleichergebnisses genügt.

Homologien der erfindungsgemäßen Cholinoxidase wurden mittels einer BLAST-Suche gegen Public-Domain-Datenbanken (Genbank am NCBI) ermittelt: gesucht wurde gegen
Database: All non-redundant GenBank CDStranslations + PDB + SwissProt + PIR + PRF

Query (Suchabfrage):
COD_Arthrobacter_nicotianae_KC2
COD_Arthrobacter_aurescens
COD_Hybrid-Protein

In allen Fällen ergab sich, daß der beste Treffer die Choline Oxidase aus Arthrobader globiformis war (siehe Tabelle 1).

Die Datenbankreferenzen der Choline Oxidase aus Arthrobacter globiformis lauten: pir∥S52489 choline oxidase (EC 1.1.3.17) - Arthrobacter globiformis pir∥S62689 choline oxidase (EC 1.1.3.17) - Arthrobacter globiformis emb|CAA59321.1| (X84895) choline oxidase

Die Aminosäureidentitäten des Gesamtproteins der Choline Oxidase aus Arthrobacter globiformis betragen zu:
- COD_Arthrobacter_nicotianae_KC2 = 62,4%
- COD_Arthrobacter_aurescens = 72,3%
- COD_Hybnd-Protein = 68,8%

Anhand der bekannten Sequenz der Cholinoxidase aus Arthrobacter globiformis wurden neue PCR-Primer (seq. 8 und 9) konstruiert.

Ein weiterer Gegenstand der vorliegende Erfindung ist somit ein Oligonukleotid, zur Verwendung als PCR-Primer, mit einer der in Seq. 8 oder Seq. 9 angegebenen Sequenzen.

Mittels PCR wurde dann eine für bakterielle Cholinoxidasen spezifischen DNA-Sonde generiert.

Ein weiterer Gegenstand der vorliegenden Erfindung ist daher eine Nukleinsäure, als DNA-Sonde, deren Nukleotidsequenz mit der in Seq. 7 angegebenen Nuldeotidsequenz zu mindestens 85 %, insbesondere mindestens 90 %, besonders bevorzugt mindestens 95 % und ganz besonders bevorzugt zu 100 % übereinstimmt.

Die erfindungsgemäßen Oligonukleotide oder Nukleinsäuren sind zur Identifizierung und/oder Gewinnung einer neuen Cholinoxidase verwendbar.

Die Herstellung einer Hybrid-Cholinoxidase kann unter Verwendung einer erfindungsgemäßen Cholinoxidase zur Fusion oder Verknüpfung mit einem anderen Protein, insbesondere zur Entwicklung eines neuen Enzyms, oder unter Verwendung einer erfindungsgemäßen Nukleinsäure zur Fusion mit einer anderen Nukleinsäure, insbesondere zur Entwicklung eines neuen Enzyms erfolgen.

Weitere Gegenstände der vorliegenden Erfindung sind:

Ein Vektor, der einen erfindungsgemäßen Nukleinsäurebereich enthält und der beispielsweise ein Klonierungsvektor oder ein Expressionsvektor sein kann. Außerdem eine Wirtszelle, die einen erfindungsgemäßen Vektor enthält Bevorzugtermaßen ist die Wirtszelle ein Bakterium, insbesondere eines, das das gebildete Protein oder Derivat ins umgebende Medium sezerniert. Die erfindungsgemäße Wirtszelle kann der Gattung Escherichia, insbesondere der Species Escherichia coli oder der Gattung Bacillus, vorzugsweise der Species Bacillus licheniformis, Bacillus amyloliquefaciens, Bacillus subtilis oder Bacillus alcalophilus oder besonders bevorzugt der-Gattung Arthrobacter und innerhalb dieser Gattung der Spezies oxidans angehören. Die Wirtszelle kann aber auch eine eukaryontische Zelle sein, insbesondere eine, die das gebildete Protein posttranslational modifiziert.

Weitere Gegenstände der vorliegenden Erfindung sind Verwendungen erfindungsgemäßen Cholinoxidasen.

Ein solcher weiterer Gegenstand der vorliegenden Erfindung ist daher die Verwendung einer Cholinoxidase, die dadurch gekennzeichnet ist, daß sie
- zur Familie der GMC-Oxidoreduktasen gehört,
- FAD als Cofaktor bindet und
- am N-terminalen Ende des Proteins, vorzugsweise an der Position 20 bis 25, einen Aminosäurebereich mit der Sequenz GxGxxG, bevorzugtermaßen mit der Sequenz GGGSAG, aufweist, wobei x für eine beliebige Aminosäure steht,
als Wasserstoffperoxid in situ erzeugendes Agens.

Eine für die erfindungsgemäße Verwendung bevorzugte Cholinoxidase ist eine der erfindungsgemäßen Cholinoxidasen.

Eine bevorzugte Ausführungsform der erfindungsgemäßen Verwendung ist die Verwendung zur Bleiche, zur Farbübertragungsinhibierung und zur Desinfektion.

Die erfindungsgemäßen Cholinoxidasen und die erfindungsgemäß verwendbaren Cholinoxidasen können vorteilhaft in Körperpflegemittel, Haarwaschmittel, Haarpflegemittel, Haarfärbe- oder Bleichmittel, Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel und Mittel zur bleichenden oder desinfizierenden Behandlung von Filtermedien, Textilien. Pelzen, Papier, Fellen oder Leder eingebracht werden.

Erfindungsgemäß bevorzugt ist ein Wasch-, oder Bleichmittel, enthaltend ein Bleichsystem, das in der Lage ist, unter Anwendungsbedingungen des Mittels Wasserstoffperoxid zu erzeugen, und gegebenenfalls synthetisches Tensid, organischen und/oder anorganischen Builder sowie sonstige übliche Bleich- oder Waschmittelbestandteile, dadurch gekennzeichnet, daß das Bleichsystem aus einer erfindungsgemäßen Cholinoxidase und einem Substrat für die Cholinoxidase besteht. Das Substrat ist vorzugsweise Cholin oder ein Cholinderivat, wie oben beschrieben.

Das erfindungsgemäße Wasch-, oder Bleichmittel weist vorzugsweise eine Oxidase-Aktivität von 1 U/g bis 20 000 U/g auf; es liegt bevorzugtermaßen als rieselfähiges Pulver mit einem Schüttgewicht von 300 g/l bis 1200 g/l, insbesondere 500 g/l bis 900 g/l, vor. Alternativ kann es aber auch in Form eines pastenförmigen oder flüssigen Waschmittels vorliegen, insbesondere in Form eines nicht-wäßrigen Flüssigwaschmittels oder einer nicht-wäßrigen Paste oder in Form eines wäßrigen Flüssiwaschmittels oder einer wasserhaltigen Paste.

Das erfindungsgemäße Wasch-, oder Bleichmittel kann in einem luftundurchlässigen Behältnis verpackt sein, aus dem es kurz vor Gebrauch oder während des Waschvorgangs freigesetzt wird, insbesondere kann die Cholinoxidase und/oder das Substrat für dieses Enzym mit einer bei Raumtemperatur oder bei Abwesenheit von Wasser für das Enzym und/oder dessen Substrat undurchlässigen Substanz umhüllt sein, welche unter Anwendungsbedingungen des Mittels durchlässig für das Enzym und/oder dessen Substrat wird.

Das erfindungsgemäße Wasch-, oder Bleichmittel enthält vorzugsweise zusätzlich zum Bleichsystem
- 5 Gew.-% bis 70 Gew.-%, insbesondere 10 Gew.-% bis 50 Gew.-% Tensid,
- 10 Gew.-% bis 65 Gew.-%, insbesondere 12 Gew.-% bis 60 Gew. -% wasserlösliches, wasserdispergierbares anorganisches Buildermaterial,
- 1 Gew.-% bis 10 Gew.-%, insbesondere 2 Gew.-% bis 8 Gew.-%, wasserlösliche organische Buildersubstanzen,
- nicht über 15 Gew.-% feste anorganische und/oder organische Säuren beziehungsweise saure Salze,
- nicht über 5 Gew.-% Komplexbildner für Schwermetalle,
- nicht über 5 Gew.-% Vergrauungsinhibitor,
- nicht über 5 Gew. -% Farbübertragungslnhibitor und
- nicht über 5 Gew.-% Schauminhibitor.

Eine erfindungsgemäße Cholinoxidase kann sowohl in Mitteln für Großverbraucher oder technische Anwender als auch in Produkten für den Privatverbraucher Anwendung finden, wobei alle im Stand der Technik etablierten Reinigungsmittelarten auch Ausführungsformen der vorliegenden Erfindung darstellen. Dazu gehören beispielsweise Konzentrate und unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegende Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz öder Leder, für solche wird nach der vorliegenden Erfindung die Bezeichnung Reinigungsmittel verwendet

Ausführungsformen der vorliegenden Erfindung umfassen alle etablierten und/oder alle zweckmäßigen Darrelchungskrmen. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Die erfindungsgemäßen Cholinoxidasen werden in erfindungsgemäßen Mitteln beispielsweise mit einzelnen oder mehreren der folgenden Inhaltsstoffe kombiniert: nichtionische, anionische und/oder kationische Tenside, (gegebenenfalls weitere) Bleichmittel, Bleichaktivatoren, Bleichkatalysatoren, Builder und/oder Cobuilder, Lösungsmittel, Verdicker, Sequestrierungsmittel, Elektrolyte, optische Aufheller, Vergrauungsinhibitoren, Korrosionsinhibitoren, Insbesondere Silberschutzmittel, Soil-Release-Wirkstoffe. Farbtransfer(oder - Übertragungs) -Inhibitoren, Schauminhibitoren, Abrasivstoffe, Farbstoffe, Duftstoffe, antimikrobielle Wirkstoffe, UV-Schutzmittel, Enzyme wie beispielsweise Proteasen, Amylasen, Lipase, Cellulasen, Hemicellulasen oder Oxidasen, Stabilisatoren, Insbesondere Enzymstabilisatoren, und andere Komponenten, die aus dem Stand der Technik bekannt sind.

Ein weiterer Gegenstand der Erfindung ist ein Oxidationsfärbemittel zum Färben von Keratinfasern, enthaltend erfindungsgemäß verwendbare Cholinoxidasen und Insbesondere erfindungsgemäße Cholinoxidasen. Als Keratinfasern sind dabei Wolle, Federn, Pelze und insbesondere menschliche Haare zu verstehen.

Zur Herstellung der erfindungsgemäßen Oxidationsmittel werden die Oxidationsfarbstoffvorprodukte sowie die Cholinoxidasen in einen geeigneten wäßrigen Träger unter Ausschluss von Luftsauerstoff eingearbeitet. Solche Träger sind z. B. verdickte wäßrige Lösungen, Cremes (Emulsionen), Gele oder tensidhaltige schäumende Zubereitungen, z. B. Shampoos oder Schaumaerosole oder andere Zubereitungen, die für die Anwendung auf dem Haar geeignet sind.

Grundsätzlich sind auch wasserfreie Pulver als Träger geeignet; in diesem Falle werden die Oxidationsfärbemittel unmittelbar vor der Anwendung in Wasser gelöst oder dispergiert. Als Trägerkomponenten werden bevorzugt
- Netz- und Emulgiermittel
- Verdickungsmittel
   Reduktionsmittel (Antioxidantien)
- haarpflegende Zusätze
- Duftstoffe und
- Lösungsmittel wie z. B. Wasser, Glycole oder niedere Alkohole eingesetzt.

Als Netz- und Emulgiermittel eignen sich z. B. anionische, zwitterionische, ampholytische und nichtionische Tenside. Auch kationische Tenside können zur Erzielung bestimmter Effekte eingesetzt werden.

Als Verdickungsmittel eignen sich die wasserlöslichen hochmolekularen Polysaccharid-Derivate oder Polypep-tide, z. B. Cellulose- oder Stärkeether, Gelatine, Pflanzengumme, Biopolymere (Xanthan-Gum) oder wasserlösliche synthetische Polymere wie z. B. Polyvinylpyrrolidon, Polyvinylalkohol, Polyethylenoxide, Polyacrylamide, Polyurethane, Polyacrylate und andere.

Weiterhin kann man tensidhaltige Zubereitungen auch durch Solubilisierung oder Emulgierung von polaren Lipiden verdicken. Solche Lipide sind z. B. Fettalkohole mit 12-18 C-Atomen, (freie) Fettsäuren mit 12-18 C-Atomen, Fettsäurepartialglyceride, Sorbitanfottsäureester, Fettsäurealkanolamide, niedrig oxethylierte Fettsäuren oder Fettalkohole, Lecithine, Sterine. Schließlich kann man gelförmige Träger auch auf Basis wässriger Seifengele, z. B. von Ammo-nium-Oleat, erzeugen.

Reduktionsmittel (Antioxidantien), die dem Träger zugesetzt werden, um eine vorzeitige oxidative Entwicklung des Farbstoffs vor der Anwendung auf dem Haar zu verhindern, sind z. B. Natriumsulfit oder Natriumascorbat.

Haarpflegende Zusätze können z.B. Fette, Öle oder Wachse in emulgierter Form, strukturgebende Additive wie z. B. Glucose oder Pyridoxin, avivierende Komponenten wie z. B. wasserlösliche Proteine, Proteinabbauprodukte, Aminosäure, wasserlösliche kationische Polymere, Silicone, Vitamine, Panthenol oder Pflanzenextrakte sein.

Schließlich können Duftstoffe und Lösungsmittel wie z. B. Glycole wie 1,2-Propylenglycole, Glycerin, Glycolether wie z. B. Butylglycol, Ethyldiglycol oder niedere einwertige Alkohole wie Ethanol oder Isopropanol enthalten sein.

Zusätzlich können noch weitere Hilfsmittel enthalten sein, die die Stabilität und Anwendungseigenschaften der Oxdationsfärbemittel verbessern, z. B. Komplexbildner wie EDTA, NTA oder Organophosphonate, Queli- und Penetrationsmittel wie z. B. Harnstoff, Guanidin, Hydrogencarbonate, Puffersalze wie z. B. Ammoniumchlorid, Ammoniumcitrat, Ammoniumsulfat oder Alkanolammoniumsalze und gegebenenfalls Treibgase.

Ein weiterer Gegenstand der Erfindung ist ein Signal-Reagenz zur Erzeugung einer Lichtemission in einem Chemilumineszenz-Assay, umfassend eine erfindungsgemäße Cholinoxidase, ein Cholinoxidase-Substrat und ein Chemilumineszenz-Reagens. Das Cholinoxidase-Substrat ist vorzugsweise Cholin. Bevorzugtermaßen ist das Chemilumineszenz-Reagens Luminol.

Chemilumineszenz-Tests sind von erheblicher Bedeutung im Bereich der Medizin und der Biowissenschaften. Besonders wichtig sind Immunotests (Immunoassays) und DNA-Sonden-Analysen. Vorteilhafterweise liefern erfindungsgemäße Cholinoxidasen, die Sauerstoff enthaltende freie Radikale (z.B. Superoxid-Anion, Hydroxyl-Radikal) und Peroxide (Hydrogenperoxid) erzeugen, bei der Reaktion mit Chemilumineszenz-Reagentien wie Lucigenin, Luminol und dessen Derivaten langlebige chemilumineszierende nachweisbare Produkte. Diese Cholinoxidase-Systeme sind von besonderem Nutzen als Tracer für den Nachwels von Analyten bei Immunoassays, Immunoblotting oder Nucleotid-Sonden-Analysen zur Bereitstellung langlebiger, Licht emittierender Einheiten nach der Reaktion mit einem Chemilumineszenz-Reagens.

Ein weiterer Gegenstand der Erfindung ist die Verwendung einer erfindungsgemäß verwendbaren Cholinoxidase und insbesondere einer erfindungsgemäßen Cholinoxidase, zur Herstellung von Betain, zur Herstellung von Lebensmitteln und/oder Lebensmittelbestandteilen sowie zur Herstellung von Tierfutter und/oder Tierfutterbestandteilen.

Betain ist als pflegender Bestandteil in Shampoos und Haarpflegemitteln sowie in Waschmitteln von Bedeutung. Außerdem wird es als Nahrungsergänzungsmittel in Tierfutter eingesetzt, da es eine protektive Wirkung auf die Leber entfaltet

Die folgenden Beispiele erläutern die Erfindung, ohne sie jedoch darauf einzuschränken:

### Beispiel 1: Mikrobiologisches Screening

Es wurden Anreicherungskulturen aus Bodenproben gewonnen. Das Auswahlkriterium war Wachstum auf Cholin als einziger C-Quelle enthaltenden Agarplatten: Unter den Kandidaten befand sich der Arthrobacter-Stamm KC2 der bei der DSMZ hinterlegt wurde (DSMZ-ID 96-878)

### Beispiel 2: Isolierung der Cholinoxidase aus dem Stamm Arthrobacter nicotianiae (KC2)

### Die Anzucht erfolgt in einem Hefemedium der folgenden Zusammensetzung:

1,0 g/L K₂HPO₄, 0,5 g/L NH₄Cl, 0,2 g/L MgSO₄ × 7 H₂O, 0,01g/L CaCl₂ × 2 H₂O, 0,8 mL Spurensalzlösung, 0,5 g/L Hefeextrakt, 5,0 g/L Cholinchlorid, 0,65 M NaCl das auf einen pH von pH = 8,0 eingestellt ist. Dielnkubation erfolgt für ca. 27 h bei 30°C und 120 rpm. Anschließend werden die Zellen durch Zentrifugation geerntet und in einer Glasperlenmühle (30% Zellsuspension) aufgeschlossen. Nach nochmaliger Zentrifugation wird er Rohextrakt, der die Cholinoxidase enthält als Überstand erhalten.

### Reinigung:

Vorbereitend wird der Rohextrakt über PD-10 Entsalzungssäulen (Amersham Pharmacia Biotech, Kat-Nr. 17-0851-01) in Puffer A (20 mM Imidazol, pH = 7,0) nach dem vom Hersteller empfohlenen Protokoll umgepuffert. Anschließend wird die Cholinoxidase-haltige Lösung auf eine lonentauschersäule (Q-Sepharose, Amersham Pharmacia Biotech, Kat-Nr. 17-1014-01) mit einem Säulenvolumen von 16,5 ml einer Fließgeschwindigkeit von 2 ml/min und unter einem Druck von 0,3 MPa aufgebracht und mit 2 Säulenvolumen Puffer A bei den gleichen Bedingungen gewaschen. Die Elution erfolgt mit 5 Säulenvolumen Puffer B (20 mM Imidazol + 1 M NaCl, pH = 7,0) durch einen linearen NaCl-Gradienten (0-100%) unter den o. g. Bedingungen.

Cholinoxidase-haltige Fraktionen werden vereinigt und Ober eine PD-10-Säule in Puffer C (20 mM Tris-HCl, pH = 7,6) nach dem vom Hersteller vorgeschlagenen Protokoll umgepuffert. Anschließend wird die Cholinoxidase-haltige Lösung auf eine Ionentauschersäule (Resource Q, Amersham Pharmacia Biotech, Kat-Nr. 17-1179-01) mit einem Säulenvolumen von 6 ml einer Fließgeschwindigkeit von 6 ml/min und unter einem Druck von 1,2 MPa aufgebracht und mit 5 Säulenvolumen Puffer C unter den gleichen Bedingungen gewaschen. Die Elution erfolgt mit 20 Säulenvolumen Puffer D (20 mM Tris-HCl + 1 M NaCl, pH = 7,6) durch einen linearen NaCl-Gradienten (0-100%) unter den o. g. Bedingungen.

### Beispiel 3: Genisolierung

Die DNA-Sonde (Seq. 6) wurde mittels PCR generiert, die Primer (Seq. 7 und 8) wurden anhand der bekannten Sequenz der Cholinoxidase aus *Arthrobacter globiformis* konstruiert, die Primer befinden sich im Gen. Die Sonde umfasst 900 bp und wurde mittels Restricktionsverdau (Fsp1 und Sty1) aus dem gewonnen DNA-Fragment geschnitten.

### Beispiel 4: Klonierung der Cholinoxidase KC2

### Sequenzen der verwendeten Primer:

Forward-Primer (Seq. 8):
KC2pETNcoF: 5' - CATGCCATGGCAAAGGGCGAAAAATTGAACATTG - 3'

Reverse-Primer (Seq. 9):
KC2pETHindR: 5' - CCCAAGCTTCTAGGCTTCGCTAACCAGTTCG - 3'

Expressionssystem: Vektor:
Vektor: pET 26 b+ von Novagen; Kat.-Nr.69862-3
Host strain: BL 21 Gold (DE 3) von Stratagene; Kat.-Nr. :230132

### Beispiel 5: Erzeugung eines Hybrid-Enzyms

Das Hybridgen besteht aus dem Cholinoxidasegen aus KC 2 und dem Cholinoxidasegen aus *Arthrobacter aurescens.* Die Genisolierung aus *Arthrobacter aurescens* erfolgte ebenfalls mit einer DNA Sonde.

### Zusammensetzung des Gens:

Base 1-269 : aus *Arthrobacter aurescens*
Base 267-1171 : aus KC 2
Base 1172-1629 : aus *Arthrobacter aurescens*

### Beispiel 6: Biochemische Charakterisierung

### Spezifische Aktivität: Aktivitätstest für Cholinoxidasen (4-Aminoantipyrin/Chromotropsäure)

### 1.Lösungen

Zur Bestimmung der Cholinoxidase-Aktivität werden folgende Lösungen benötigt:

| | | | | |
|---|---|---|---|---|
| • | Peroxidreagenz: | K₂HPO₄ | 75 mM | 0,68g/50mL |
| | | NaH₂PO₄ | 125 mM | 0,89g/50mL |
| | | Chromotropsäure | 12,5mM | 0,2503g/50mL |
| | | 4-Aminoantipyrin | 0,6mM | 6mg/50mL |
| | | | | |
| • | Phosphatpuffer: | K₂HPO₄ | 75mM | 13,6g/L |
| | pH=6,5 | NaH₂PO₄ | 125mM | 17,8g/L |
| | | | | |
| • | Peroxidase: | 52 U/mL Peroxidase aus Meerrettich in Phosphat-Puffer | | |
| | | | | |
| • | Davis-Puffer: | Citronensäure x 1H₂O | 0,1M | 21,01g/L |
| | Stammlösung | NaB₄O₇ x 1H₂O | 0,1M | 19,07g/L |
| | | KCl | 0,1M | 7,46g/L |
| | | KH₂PO₄ | | 13,61g/L |
| | | Tris-(hydroxymethyl) -aminomethan | 0,1M | 12,11g/L |
| | | (1:4 mit dest. H₂O verdünnen und mit 0,4M NaOH auf pH 9,5 einstellen) | | |
| | | | | |
| • | 20 mM NaN₃: | 65mg/50mL In Davis-Puffer pH=9,5 | | |
| | | | | |
| • | 250 mM Cholinchlorid in dest. H₂O | | | |

### 2. Durchführung:

Zu 150 µl Cholinoxidase-haltiger Lösung werden 30 µL 20 mM NaN₃-Lösung, 45 µL Davis-Puffer pH=9,5 und150 µL 250 mM Cholinchlorid gegeben und der Ansatz nach Mischen für 30 min bei 800 rpm und 37°C inkubiert. Anschließend werden 525 µL Peroxidreagenz und75 µL Peroxidaselösung zugegeben und bei Raumtemperatur für 5 min inkubiert. Schließlich wird die Absorption der Lösung bei einer Wellenlänge von □=600 nm bestimmt.

Die Bestimmung der Aktivität erfolgt durch den Vergleich mit einer Kalibrationskurve, die mit einer geeigneten Verdünnungsreihe von Wasserstoffperoxid in 20 mM Phosphatpuffer (pH 6,5) unter den oben beschriebenen Bedingungen erstellt wurde.

Die Proteinmenge pro Probenvolumen wird mit dem BCA Protein Assay Kit (Pierce Biotechnology, Kat-Nr. 23227) nach dem von Hersteller vorgeschlagenen Protokoll bestimmt und in mg/µl angegeben. Die spezifische Aktivität bezeichnet die Aktivität pro mg Protein und wird angegeben in U/mg bzw. mU/mg = 10⁻³ U/mg Die spezifische Aktivität des KC 2 bezieht sich hier auf das Probevolumen im Aktivitätstest und beträgtim Rohextrakt (mechanischer Aufschluß) 41 mU/mg und nach Aufreinigung
123 mU/mg

### pH-Stabilität:

Die Cholinoxidase ist in einem pH-Bereich von pH 6-12 stabil, insbesondere zwischen pH 8-10.

### Durchführung:

Die aufgereinigte Oxidase wird 1:30 mit Davis-Puffer des entsprechenden pH-Werts verdünnt und für eine Stunde bei 30°C und 800 rpm inkubiert. Anschließend werden mit 150 µl der Lösung ein Aktivitätstest wie oben beschrieben durchgeführt.

### Temperaturprofil:

Die Cholinoxidase im Temperaturbereich von 10 °C bis 70 °C und besonders im Temperaturbereich von 25 °C bis 40 °C aktiv. Sie ist ein dem Temperaturbereich von 10 °C bis 50 °C und besonders im Temperaturbereich von 10 °C bis 40 °C stabil.

### Durchführung:

Zur Bestimmung der Aktivität bei einer bestimmten Temperatur wird die aufgereinigte Cholinoxidase in Davis-Puffer pH 9,5 mit Natriumazid (3 mM Endkonzentration entsprechend verdünnt Die Aktivitätsbestimmung erfolgt mit 150 µl Lösung analog dem oben beschriebenen Protokoll mit dem Unterschied, dass anstatt bei 37 °C bei der entsprechenden Temperatur inkubiert wird.
Zur Bestimmung der Temperaturstabilität wird die aufgereinigte Cholinoxidase entsprechend verdünnt in Davis-Puffer pH 9,5 mit Natriumazid (3 mM Endkonzentration) bei der entsprechenden Temperatur für eine Stunde vorinkubiert.Anschließend erfolgt ein Aktivitätstest mit 150 µl der Lösung nach obigem Protokoll.

### Beispiel 7: Erzeugung von Mutanten mit verbesserter spezifischer Aktivität gegenüber Cholin

Die KC2 wurde in E. coli exprimiert, wozu ein Konstrukt mit Seq. 17 erzeugt wurde. Sie unterscheidet sich durch die Mutationen C2T, A4G und G5C am Genanfang und der stillen Mutation C1186A. Dadurch unterscheiden sich Seq. 1 und Seq. 10 in dem Aminosäuren T1M und R2A.
Die Mutanten sind Varianten der Cholinoxidase KC2 in E. coli (Seq. 17) und wurde im gleichen Konstrukt exprimiert.
Die Mutation C1186A ist eine stille Mutation, die in Arthrobacter nicht zu finden ist. Sie tritt aber in dem in E. coli exprimierten Cholinoxidasegenen (KC2 in E. coli, KC2 M85V, KC2 A27V, KC2 M85V2 d. h. Seq. 17,18,19, 20) auf.
Die Mutation C612T in der Mutante KC2 M85V2 (Seq. 20) ist ebenfalls eine stille Mutation, die aber zu einer verbesserten Expression führt. In der Fachliteratur wird ein solches Phänomen Expressionsmutante genannt. Im vorliegenden Fall ist die verbesserte Expression von KC2 M85V2 an der höheren spezifischen Aktivität im Rohextrakt zu erkennen.

Das Gen KC2s (Seq. 21) wurde aus Seq. 10 erhalten, indem die ersten 18 Basen von Gen KC2 (Seq. 17) abgeschnitten wurden. Zur Klonierung wurde Seq. 22 erzeugt, die sich in Base 1 (T1A) von Seq. 21 unterscheidet Das daraus resultierende Genprodukt ist in Seq. 15 dargestellt.

In gleicher Weise kann von jeder genannten Mutante eine "KC2s-Variante" hergestellt werden.

### Zusammenfassung der Mutanten und ihrer Eigenschaften:

| Cholinoxidase | Spezifische Aktivität im Rohextrakt | Spezifische Aktivität, Gereinigt | Mutation, DNA-Ebene | Mutation, Proteinebe ne |
|---|---|---|---|---|
| KC2 aus Arthrobacter (Seq. 1 und Seq. 4) | ND | ND | - | - |
| KC2, heterolog aus *E. coli,* (Seq. 10 und Seq 17) | 80,6 mU/mg | 123 mU/mg | C1186A | - |
| KC2 M85V (Seq. 11 und Seq. 18) | 112,6 mU/mg | 870 mU/mg | A253G, C1186A, C1188A | M85V |
| KC2 A27V (Seq. 12 und 19) | 132,6 mU/mg | 670 mU/mg | C80T, C1186A, C1254T, C1369T | A27V |
| KC2 M85V2 (Seq. 13 und 20) | 213,9 mU/mg | 920 mU/mg | A253G, C612T, C1186A, 1188A | M85V |
| KC2s (Seq. 15 und Seq. 22) | 70 mU/mg | 508 mU/mg | C1186A | - |

### Beispiel 8: Erzeugung von KC2s (KC2s)

Sequenzen der verwendeten Primer
KC2spETNF: 5' TTCCATATGAACATTGAAAAGAAGGACTTCGACTACATTG 3' (Seq. 23)
KC2pETHR (Seq. 9)

Die Amplifizierung aus der genomischen DNA von Arthrobacter nicotianae oder aus dem Gen der Cholinoxidase KC2 erfolgte mittels geeigneter PCR-Methode.

Die Klonierung erfolgte in NdeI-HindIII-Site des Vektors pET 26 b+ von Novagen (Kat-Nr. 69862-3).
Die Transformation erfolgte mittels Standardmethoden in *E. coli* BL21 Gold (DE3) (Stratagene, Kat.-Nr. 230132)

### Beispiel 9: Waschversuch

### Waschmatrix: Rahmenrezeptur, wie in der nachfolgenden Tabelle dargestellt:

| Chem. Name | Rahmenrezeptur |
|---|---|
| | (% Reinsubstanz) |
| Xanthan-Gum | 0,3- 0,5 |
| Antischaummittel | 0,2-0,4 |
| Glycerin | 6-7 |
| Ethanol | 0,3-0,5 |
| FAEOS | 4-7 |
| Nonionisches Tensid (FAEO, APG, u.a.) | 24 - 28 |
| | |
| Borsäure | 1 |
| Sodium citrat x 2H2O | 1-2 |
| Caustic soda | 2-4 |
| Kokosnußfettsäure | 14-16 |
| HEDP | 0,5 |
| PVP | 0-0,4 |
| optische Aufheller | 0-0,05 |
| Farbe | 0-0,001 |
| Parfüm | 0-2 |
| H2O, demin. | Rest |

### Dosierung: 4,4 g/l

Anschmutzung: Tee auf Baumwolle Tee auf Baumwolle, z. B. Anschmutzung 020 J Co von wfk Testgewebe GmbH (Brüggen-Bracht, Deutschland), E-167 von EMPA Testmaterialien AG (St. Gallen, Schweiz) oder HTB von Henkel KGaA (Düsseldorf, Deutschland).
Zeit 30 min
pH während des Vorgangs: 10 (mit Soda eingestellt)
Auswertung: Bestimmung des L-Wertes

### Durchführung:

Testgewebe (Durchmesser 10 mm) wurden in einer 24 well Mikrotiterplatte in 1 mL Waschlauge für 30 min bei 37 °C und einer Schüttelfrequenz von 100 rpm inkubiert. Die Waschlauge enthielt 265 mU Cholinoxidase KC2 oder proteinäquivalente Mengen der Mutanten bzw. KC2s aus den entsprechenden Präparationen. Die Substratkonzentration betrug zwischen 100 mM und 200 mM Cholinchlorid. Während der Inkubation wurde Luft durch eine Kanüle mit einem Innendurchmesser von 0,5 bis 1 mm in jeden Versuchansatz eingeleitet. Jeder Versuch wurde als Doppelbestimmung gegen eine doppelt bestimmte Kontrolle ohne Cholinoxidase durchgeführt.

Nach dem Waschen wurde der Weißheitsgrad der gewaschenen Textilien im Vergleich zu einem Weißstandard (d/8, ∅ 8mm, SCI/SCE) gemessen, der auf 100% normiert worden war. Die Messung erfolgte an einem Farbmessgerät (Minolta Cm508d) mit einer Lichtartelnstellung von 10°/D65. Die erhaltenen Ergebnisse werden als Prozent Remission, das heißt als Prozentangabe im Vergleich zum Weißstandard zusammen mit den jeweiligen Anfangswerten in folgender Tabelle zusammengestellt. Die Bleichleistung ergibt sich als ΔL der Differenz der Remissionswerte von Basiswaschmittel mit Cholinoxidase und Kontrolle ohne Cholinoxidase unter gleichen Versuchsbedingungen.

### Einfluss von Bleichaktivatoren

| Basiswaschmittel mit: | TAED-Konzentration Gew% | Cholinkonzentration mmol/l | Bleichleistung ΔL | Standardabweichung |
|---|---|---|---|---|
| KC2 | 0 | 200 | 3,40 | 0,28 |
| KC2 | 0,03 | 200 | 4,50 | 0,07 |

Man erkennt, dass die erfindungsgemäße Cholinoxidase KC2 in der Basisrezeptur ohne TAED einen bleichenden Effekt auf die getestete Anschmutzung hat. Dieser Effekt wird durch die Zugabe von TAED verstärkt.

### Waschleistung von Mutanten gegenüber KC2 und KC2s

| Basiswaschmittel mit: | TAED-Konzentration Gew% | Cholinkonzentration mmol/l | Bleichleistung ΔL | Standardabweichung |
|---|---|---|---|---|
| KC2 | 0,03 | 100 | 3,34 | 0,23 |
| KC2 M85V (M85V) | 0,03 | 100 | 4,26 | 0,55 |
| KC2 A27V (A27V) | 0,03 | 100 | 4,34 | 0,59 |
| KC2 M85V2 (M85V) | 0,03 | 100 | 4,43 | 0,46 |
| KC2s | 0,03 | 100 | 3,57 | 0,32 |

Die erfindungsgemäßen Mutanten der Cholinoxidase KC2 (KC2 M85V, KC2 A27V und KC2 M85V2) sowie KC2s zeigen eine ausgeprägtere Bleichleistung als KC2 gegenüber Teeanschmutzungen in einer Basisrezeptur mit TAED.

### Beispiel 10: Vergleich der Bleichleistung von Cholinoxidase mit einem herkömmlichen Bleichsystem.

Waschmatrix: Wie in Beispiel 9
Dosierung: 4,4 g/l
Anschmutzung: Tee auf Baumwolle, z. B. Anschmutzung 020 J Co von wfk Testgewebe GmbH (Brüggen-Bracht, Deutschland), E-167 von EMPA Testmaterialien AG (St. Gallen, Schweiz) oder HTB von Henkel KGaA (Düsseldorf, Deutschland).
Zeit: 30 min
pH während des Vorgangs: 10 (mit Soda eingestellt)
Auswertung: Bestimmung des L-Wertes

### Durchführung:

Testgewebe (Durchmesser 10 mm) wurden in einer 24 well Mikrotiterplatte in 1 mL Waschlauge für 30 min bei 37 °C und einer Schüttelfrequenz von 100 rpm inkubiert. Die Waschlauge enthielt 265 mU Cholinoxidase KC2 und 200 mM Cholinchlorid. Während der Inkubation wurde Luft durch eine Kanüle mit einem Innendurchmesser von 0,5 bis 1 mm In jeden Versuchansatz eingeleitet. Zur Bestimmung der Bleichleistung von chemischer Bleiche wurden Ansätze mit Waschlauge kurz vor dem Versuch 97 mg Percarbonat durchgeführt. Somit wurde ein vorzeitiger Zerfall des Percarbonats im Testansatz ausgeschlossen Jeder Versuch wurde als Doppelbestimmung gegen eine doppelt bestimmte Kontrolle ohne Cholinoxidase bzw. Percarbonat durchgeführt.
Nach dem Waschen wurde der Weißheitsgrad der gewaschenen Textilien im Vergleich zu einem Weißstandard (d/8, ∅ 8mm, SCI/SCE) gemessen, der auf 100% normiert worden war. Die Messung erfolgte an einem Farbmessgerät (Minolta Cm508d) mit einer Lichtarteinstellung von 10°/D65. Die erhaltenen Ergebnisse werden als Prozent Remission, das heißt als Prozentangabe im Vergleich zum Weißstandard zusammen mit den jeweiligen Anfangswerten in folgender Tabelle zusammengestellt. Die Bleichleistung ergibt sich als □L der Differenz der Remissionswerte von Basiswaschmittel mit Cholinoxidase und Kontrolle ohne Cholinoxidase unter gleichen Versuchsbedingungen.

### Vergleich enzymatischer mit chemischer Bleiche

| Basiswaschmittel mit: | TAED-Konzentration Gew% | Cholinkonzentration mmol/l | Bleichleistung ΔL | Standardabweichung |
|---|---|---|---|---|
| Percarbonat | 0,03 | 0 | 3,03 | 0,30 |
| KC2 | 0,03 | 200 | 4,50 | 0,28 |

Man erkennt, dass die erfindungsgemäße Cholinoxidase KC2 in der Basisrezeptur mit TAED einen stärker bleichenden Effekt auf die getestete Anschmutzung hat als eine standardmäßig eingesetzte chemische Bleiche.

### Beispiel 11: Vergleich der Bleichleistung von Cholinoxidase an verschiedenen bleichbaren Anschmutzungen.

Waschmatrix: Wie in den Beispielen 9 und 10
Dosage: 4,4 g/l
Anschmutzung:
Tee auf Baumwolle, z. B. Anschmutzung 020 J Co von wfk Testgewebe GmbH (Brüggen-Bracht, Deutschland), E-167 von EMPA Testmaterialien AG (St. Gallen, Schweiz) oder HTB von Henkel KGaA (Düsseldorf, Deutschland).
Brombeere auf Baumwolle, z. B. Anschmutzung 041 BB Co von wfk Testgewebe GmbH (Brüggen-Bracht, Deutschland), oder HBBB von Henkel KGaA (Düsseldorf, Deutschland).
Rotwein auf Baumwolle, z. B. E-114 von EMPA Testmaterialien AG (St. Gallen, Schweiz) oder HRB von Henkel KGaA (Düsseldorf, Deutschland).
Zeit: 30 min
pH während des Vorgangs: 10 (mit Soda eingestellt)
Auswertung: Bestimmung des L-Wertes

### Durchführung:

Testgewebe (Durchmesser 10 mm) wurden in einer 24 well Mikrotiterplatte in 1 mL Waschlauge für 30 min bei 37 °C und einer Schüttelfrequenz von 100 rpm inkubiert. Die Waschlauge enthielt 265 mU Cholinoxidase KC2 und 200 mM Cholinchlorid. Während der Inkubation wurde Luft durch eine Kanüle mit einem Innendurchmesser von 0,5 bis 1 mm in jeden Versuchansatz eingeleitet. Jeder Versuch wurde für jede Anschmutzung als Doppelbestimmung gegen eine doppelt bestimmte Kontrolle ohne Cholinoxidase durchgeführt.
Nach dem Waschen wurde der Weißheitsgrad der gewaschenen Textilien im Vergleich zu einem Weißstandard (d/8, ∅ 8mm, SCI/SCE) gemessen, der auf 100% normiert worden war. Die Messung erfolgte an einem Farbmessgerät (Minolta Cm508d) mit einer Lichtarteinstellung von 10°/D65. Die erhaltenen Ergebnisse werden als Prozent Remission, das heißt als Prozentangabe im Vergleich zum Weißstandard zusammen mit den jeweiligen Anfangswerten in folgender Tabelle zusammengestellt. Die Bleichleistung ergibt sich als ΔL der Differenz der Remissionswerte von Basiswaschmittel mit Cholinoxidase und Kontrolle ohne Cholinoxidase unter gleichen Versuchsbedingungen an den jeweiligen Anschmutzungen.

### Vergleich enzymatischer Bleiche and verschiedenen Anschmutzungen

| Basiswaschmittel mit: | TAED-Konzentration Gew% | Anschmutzung | Bleichleistung ΔL | Standardabweichung |
|---|---|---|---|---|
| KC2 | 0,03 | Tee | 4,50 | 0,28 |
| KC2 | 0,03 | Brombeere | 1,40 | 0,35 |
| KC2 | 0,03 | Rotwein | 2,00 | 0,14 |

Man erkennt, dass die erfindungsgemäße Cholinoxidase KC2 unter den angegebenen Bedingungen einen bleichenden Effekt auf die relevanten Anschmutzungen hat

### Sequenzen:

Seq. 1 (Cholinoxidase aus Arthrobacter nicotianiae (KC2, DSMZ-ID 96-878))
Seq. 2 Cholinoxidase aus Arthrobacter aurescens (ATCC 13344)
Seq. 3 (Hybride Cholinoxidase)
Seq. 4 (Nukleinsäuresequenz, Cholinoxidase aus Arthrobacter nicotianiae (KC2, DSMZ-ID 96-878))
Seq. 5 Nukleinsäuresequenz, (Cholinoxidase aus Arthrobacter aurescens ATCC 13344)
Seq. 6 (Nukleinsäuresequenz, hybride Cholinoxidase)
Seq. 7 (Nukleinsäuresequenz, Sonde zur Isolierung einer Cholinoxidase)
Seq. 8 (Nukleinsäuresequenz, Primer zur Klonierung einer Cholinoxidase)
   5' - CATGCCATGGCAAAGGGCGAAAAATTGAACATTG - 3'
Seq. 9 (Nukleinsäuresequenz, Primer zur Klonierung einer Cholinoxidase)
   5' - CCCAAGCTTCTAGGCTTCGCTAACCAGTTCG - 3'

### Tabellen:

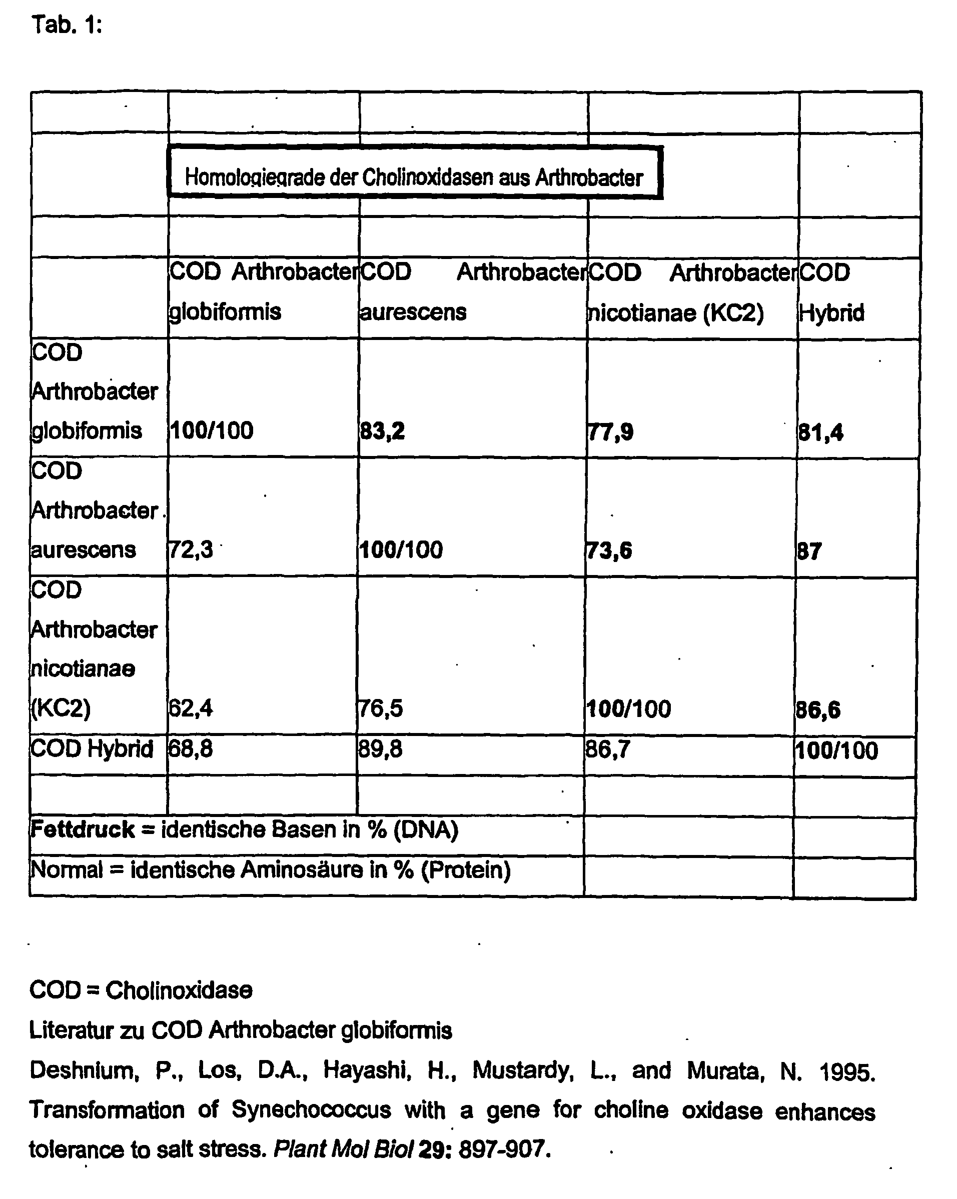

.

## Patentansprüche

1. Cholinoxidase, deren Aminosäuresequenz mit der in Seq. 1 angegebenen
Aminosäuresequenz übereinstimmt,
oder deren Aminosäuresequenz mit der in Seq. 1 angegebenen Aminosäuresequenz zu mindestens 85% übereinstimmt und die aus einer Cholinoxidase, deren
Aminosäuresequenz mit der in Seq. 1 angegebenen Aminosäuresequenz
übereinstimmt, durch ein oder mehrfachen konservativen Aminosäureaustausch erhältlich ist.

2. Cholinoxidase, deren Aminosäuresequenz mit einer der in Seq. 10 bis Seq. 15 angegebenen Aminosäuresequenzen zu 100 % übereinstimmt.

3. Nukleinsäure, kodierend für eine Cholinoxidase, gemäß einem der Ansprüche 1 oder 2 deren Aminosäuresequenz einen Teil enthält, der mit einer der in Seq. 1 oder 10 bis 15 angegebenen Amino-säuresequenzen zu mindestens 90 %, besonders bevorzugt mindestens 95 % und ganz besonders bevorzugt zu 100 % identisch ist.

4. Für eine Cholinoxidase gemäß einem der Ansprüche 1 oder 2 kodierende Nukleinsäure, deren Nukleotidsequenz mit der in Seq. 4 angegebenen Nukleotidsequenz zu mindestens 85% übereinstimmt.

5. Für eine Cholinoxidase gemäß einem der Ansprüche 1 oder 2 kodierende Nukleinsäure, deren Nukleotidsequenz mit der in Seq. 4 angegebenen Nukleotidsequenz zu mindestens 90 %, besonders bevorzugt mindestens 95 % und ganz besonders bevorzugt zu 100% übereinstimmt.

6. Für eine Cholinoxidase kodierende Nukleinsäure, deren Nukleotidsequenz mit einer der in Seq. 17 bis 22 angegebenen Nukleotidsequenzen zu 100 % übereinstimmt.

7. Oligonukleotid mit einer der in Seq. 8 oder Seq. 9 angegebenen Sequenzen zur Verwendung als PCR-Primer.

8. Für eine bakterielle Cholinoxidase spezifische DNA-Sonde, deren Nukleotidsequenz mit der in Seq. 7 angegebenen Nukleotidsequenz zu mindestens 85 %, insbesondere mindestens 90 %, besonders bevorzugt mindestens 95 % und ganz besonders bevorzugt zu 100 % übereinstimmt

9. Verwendung eines Oligonukleotids nach Anspruch 7 oder einer Nukleinsäure nach Auspruch 8 zur Identifizierung und/oder Gewinnung einer Cholinoxidase.

10. Verwendung einer Cholinoxidase nach einem der Ansprüche 1 oder 2 zur Fusion oder Verknüpfung mit einem anderen Protein, insbesondere zur Entwicklung eines neuen Enzyms.

11. Verwendung einer Nukleinsäure nach einem der Ansprüche 3 bis 6 zur Fusion mit einer anderen Nukleinsäure, insbesondere zur Entwicklung eines neuen Enzyms.

12. Vektor, der einen der in den Ansprüchen 3 bis 6 bezeichneten Nukleinsäurebereiche enthält.

13. Klonierungsvektor gemäß Anspruch 12.

14. Expressionsvektor gemäß Anspruch 12.

15. Wirtszelle, enthaltend einen Vektor nach einem der Ansprüche 12-14, wobei die Wirtszelle ein Mikroorganismus ist.

16. Wirtszelle gemäß Anspruch 15, **dadurch gekennzeichnet, daß** sie ein Bakterium ist, insbesondere eines, das das gebildete Protein ins umgebende Medium sezerniert.

17. Wirtszelle gemäß Anspruch 16, **dadurch gekennzeichnet, daß** sie der Gattung Escherichia, insbesondere der Species Escherichia coli angehört.

18. Wirtszelle gemäß Anspruch 15, **dadurch gekennzeichnet, daß** sie der Gattung Bacillus, vorzugsweise der Species Bacillus licheniformis, Bacillus amyloliquefaciens, Bacillus subtilis oder Bacillus alcalophilus angehört.

19. Wirtszelle gemäß Anspruch 15, **dadurch gekennzeichnet, daß** sie besonders bevorzugt der Gattung Arthrobacter und innerhalb dieser Gattung der Spezies oxidans angehört.

20. Wirtszelle gemäß Anspruch 15, **dadurch gekennzeichnet, daß** sie eine eukaryontische Zelle ist, insbesondere eine, die das gebildete Protein posttranslational modifiziert.

21. Verwendung einer Cholinoxidase nach einem der Ansprüche 1 oder 2 als Wasserstoffperoxid in situ erzeugendes Agens.

22. Verwendung nach Anspruch 24, **dadurch gekennzeichnet, daß** die Cholinoxidase den Aminosäurebereich mit der Sequenz GxGxxG an der Position 20 bis 25 aufweist.

23. Verwendung nach Anspruch 22, **dadurch gekennzeichnet, daß** die Sequenz GxGxxG GGGSAG bedeutet.

24. Verwendung einer Cholinoxidase gemäß der Definition in einem der Ansprüche 21 bis 23 zur Bleiche, zur Farbübertragungsinhibierung und zur Desinfektion.

25. Körperpflegemittel, Haarwaschmittel, Haarpflegemittel, Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel oder Mittel zur bleichenden oder desinfizierenden Behandlung von Filtermedien, Textilien, Pelzen, Papier, Fellen oder Leder, enthaltend eine Cholinoxidase gemäß einem der Ansprüche 1 oder 2.

26. Wasch- oder Bleichmittel, enthaltend ein Bleichsystem, das in der Lage ist, unter Anwendungsbedingungen des Mittels Wasserstoffperoxid zu erzeugen, und gegebenenfalls synthetisches Tensid, organischen und/oder anorganischen Builder sowie sonstige übliche Bleich- oder Waschmittelbestandteile, insbesondere Bleichaktivatoren, vorzugsweise TEAD, **dadurch gekennzeichnet, daß** das Bleichsystem aus einer Cholinoxidase gemäß einem der Ansprüche 1 oder 2 und einem Substrat für die Cholinoxidase besteht.

27. Mittel nach Anspruch 26, **dadurch gekennzeichnet, daß** das Substrat Cholin, Betainaldehyd oder ein Cholinderivat ist.

28. Mittel nach Anspruch 26 oder 27, **dadurch gekennzeichnet, daß** es eine Oxidase-Aktivität von 3 U/g bis 20 000 U/g aufweist.

29. Mittel nach einem der Ansprüche 26 bis 28, **dadurch gekennzeichnet, daß** es als rieselfähiges Pulver mit einem Schüttgewicht von 300 g/l bis 1200 g/l, insbesondere 500 g/l bis 900 g/l, vorliegt.

30. Mittel nach einem der Ansprüche 26 bis 28, **dadurch gekennzeichnet, daß** es in Form eines pastenförmigen oder flüssigen Waschmittels vorliegt

31. Mittel nach einem der Ansprüche 26 bis 28 oder 30, **dadurch gekennzeichnet, daß** es in Form eines nicht-wäßrigen Flüssigwaschmittels oder einer nicht-wäßrigen Paste vorliegt.

32. Mittel nach einem der Ansprüche 26 bis 28, 30 oder 31, **dadurch gekennzeichnet, daß** es in Form eines wäßrigen Flüssigwaschmittels oder einer wasserhaltigen Paste vorliegt und in einem luftundurchlässigen Behältnis verpackt ist, aus dem es kurz vor Gebrauch oder während des Waschvorgangs freigesetzt wird.

33. Mittel nach einem der Ansprüche 26 bis 32, **dadurch gekennzeichnet, daß** die Cholinoxidase und/oder das Substrat für dieses Enzym mit einer bei Raumtemperatur oder bei Abwesenheit von Wasser für das Enzym und/oder dessen Substrat undurchlässigen Substanz umhüllt ist, welche unter Anwendungsbedingungen des Mittels durchlässig für das Enzym und/oder dessen Substrat wird.

34. Mittel nach einem der Ansprüche 26 bis 33, **dadurch gekennzeichnet, daß** es zusätzlich zum Bleichsystem
• 5 Gew.-% bis 70 Gew.-%, insbesondere 10 Gew.-% bis 50 Gew.-% Tensid,
• 10 Gew.-% bis 65 Gew.-%, insbesondere 12 Gew.-% bis 60 Gew.-% wasserlösliches, wasserdispergierbares anorganisches Buildermaterial,
• 1 Gew.-% bis 10 Gen.-%, insbesondere 2 Gew.-% bis 8 Gen.-%, wasserlösliche organische Buildersubstanzen,
• nicht über 15 Gew.-% feste anorganische und/oder organische Säuren beziehungsweise saure Salze,
• nicht über 5 Gen.-% Komplexbildner für Schwermetalle,
• nicht über 5 Gew.-% Vergrauungsinhibitor,
• nicht über 5 Gew. -% Farbübertragungsinhibitor und
• nicht über 5 Gew.-% Schauminhibitor
enthält.

35. Oxidationsfärbemittel zum Färben von Keratinfasern, insbesondere von Haaren, enthaltend Cholinoxidasen gemäß einem der Ansprüche 1 oder 2.

36. Signal-Reagenz zur Erzeugung einer Lichtemission in einem Chemilumineszenz-Assay, umfassend eine Cholinoxidase gemäß einem der Ansprüche 1 oder 2, ein Cholinoxidase-Substrat und ein Chemilumineszenz-Reagens.

37. Signal-Reagenz nach Anspruch 36, worin das Cholinoxidase-Substrat ausgewählt ist unter Cholin, Cholinderivaten und Betainaldehyd.

38. Signal-Reagenz nach Anspruch 36 oder 37, worin das Chemilumineszenz-Reagens Luminol ist.

39. Verwendung einer Cholinoxidase gemäß einem der Ansprüche 1 oder 2 zur Herstellung von Betain.

40. Verwendung einer Cholinoxidase gemäß einem der Ansprüche 1 oder 2 zur Herstellung von Lebensmitteln und/oder Lebensmiffelbestandteifen.

41. Verwendung einer Cholinoxidase gemäß einem der Ansprüche 1 oder 2 zur Herstellung von Tierfutter und/oder Tierfutterbestandteifen.

## Claims

1. Choline oxidase, the amino-acid sequence of which coincides with the amino-acid sequence indicated in Seq. 1,
or the amino-acid sequence of which coincides to within at least 85% with the amino-acid sequence indicated in Seq. 1, and which may be obtained from a choline oxidase, the amino-acid sequence of which coincides with the amino-acid sequence indicated in Seq. 1, by means of one or more conservative amino-acid exchanges.

2. Choline oxidase, the amino acid sequence of which coincides with one of the amino-acid sequences indicated in Seq. 10 to Seq.15 to within 100%.

3. A nucleic acid, coding for a choline oxidase according to any of claims 1 or 2, the amino-acid sequence of which contains a portion which is identical with one of the amino-acid sequences indicated in Seq. 1 or 10 to 15 to within at least 90%, more preferably to within at least 95% and most preferably to within 100%.

4. A nucleic acid, coding for a choline oxidase according to any of claims 1 or 2, the nucleotide sequence of which coincides with the nucleotide sequence indicated in Seq. 4 to within at least 85%.

5. A nucleic acid, coding for a choline oxidase according to any of claims 1 or 2, the nucleotide sequence of which coincides with the nucleotide sequence indicated in Seq. 4 to within at least 90%, more preferably to within at least 95%, and most preferably to within 100%.

6. A nucleic acid, coding for a choline oxidase, the nucleotide sequence of which coincides with one of the nucleotide sequences indicated in Seq. 17 to 22, to within 100%.

7. An oligonucleotide with one of the sequences indicated in Seq. 8 or Seq. 9 for use as a PCR primer.

8. A DNA probe specific for a bacterial choline oxidase, the nucleotide sequence of which coincides with the nucleotide sequence indicated in Seq. 7 to within at least 85%, in particular at least 90%, more preferably at least 95% and most preferably to within 100%.

9. Use of an oligonucleotide according to claim 7 or of a nucleic acid according to claim 8, for identifying and/or obtaining a choline oxidase.

10. Use of a choline oxidase according to any of claims 1 or 2 for fusion or coupling with another protein, in particular for developing a novel enzyme.

11. Use of a nucleic acid according to any of claims 3 to 6 for fusion with another nucleic acid, in particular for developing a novel enzyme.

12. A vector, which contains one of the nucleic acid domains designated in claims 3 to 6.

13. The cloning vector according to claim 12.

14. The expression vector according to claim 12.

15. A host cell containing a vector according to any of claims 12 to 14, wherein the host cell is a microorganism.

16. The host cell according to claim 15, **characterized in that** it is a bacterium, in particular one which secretes the formed protein in the surrounding medium.

17. The host cell according to claim 16, **characterized in that** it belongs to the genus *Escherichia,* in particular to the species *Escherichia coli.*

18. The host cell according to claim 15, **characterized in that** it belongs to the genus Bacillus, preferably to the species *Bacillus licheniformis, Bacillus amyloliquefaciens, Bacillus subtilis* or *Bacillus alcalophilus.*

19. The host cell according to claim 15, **characterized in that** it more preferably belongs to the genus *Arthrobacter* and to the species *oxidans* within this genus.

20. The host cell according to claim 15, **characterized in that** it is a eukaryotic cell, in particular one which modifies post-translationally the formed protein.

21. Use of a choline oxidase according to any of claims 1 or 2 as an agent producing hydrogen peroxide *in situ.*

22. Use according to claim 21, **characterized in that** the choline oxidase has the amino-acid domain with the sequence GxGxxG in position 20 to 25.

23. Use according to claim 22, **characterized in that** the sequence GxGxxG represents GGGSAG.

24. Use of a choline oxidase according to the definition in any of claims 21 to 23 for bleaching, for inhibiting color transfer and for disinfection.

25. Body care agents, hair washing agents, hair care agents, buccal, dental or dental prostheses care agents, cosmetics, washing agents, cleaning agents, rinsing agents, hand washing agents, hand dishwashing agents, machine dishwashing agents, disinfection agents, or agents for bleaching or disinfecting treatment of filter media, textiles, furs, paper, skins, or leather, containing a choline oxidase according to any of claims 1 or 2.

26. Washing or bleaching agent containing a bleaching system which is able to produce hydrogen peroxide under application conditions of the agent and optionally a synthetic surfactant, an organic and/or inorganic builder as well as other common bleaching or washing agent components, in particular bleaching activators, preferably TEAD, **characterized in that** the bleaching system consists of a choline oxidase according to any of claims 1 or 2, and of a substrate for the choline oxidase.

27. The agent according to claim 26, **characterized in that** the substrate is choline, betaine aldehyde or a choline derivative.

28. The agent according to claim 26 or 27, **characterized in that** it has an oxidase activity from 3 U/g to 20,000 U/g.

29. The agent according to any of claims 26 to 28, **characterized in that** it exists as flowable powder with an apparent density from 300 g/L to 1,200 g/L, in particular 500 g/L to 900 g/L.

30. The agent according to any of claims 26 to 28, **characterized in that** it exists in the form of a pasty or liquid washing agent.

31. The agent according to any of claims 26 to 28 or 30, **characterized in that** it exists in the form of a non-aqueous liquid washing agent or a non-aqueous paste.

32. The agent according to any of claims 26 to 28, 30 or 31, **characterized in that** it exists in the form of an aqueous liquid washing agent or a water-containing paste and is packed in an airproof container, out of which it is released shortly before use or during the washing process.

33. The agent according to any of claims 26 to 32, **characterized in that** the choline oxidase and/or the substrate for this enzyme is coated with an impervious substance for the enzyme and/or its substrate at room temperature or in the absence of water, which, under application conditions of the agent, becomes pervious for the enzyme and/or its substrate.

34. The agent according to any of claims 26 to 33, **characterized in that** in addition to the bleaching system, it contains
• 5% by weight to 70% by weight, in particular 10% by weight to 50% by weight of surfactant,
• 10% by weight to 65% by weight, in particular 12% by weight to 60% by weight of a water-soluble, water-dispersible inorganic builder material,
• 1% by weight to 10% by weight, in particular 2% by weight to 8% by weight of water-soluble organic builder substances,
• not more than 15% by weight of solid inorganic and/or organic acids or acid salts,
• not more than 5% by weight of complexing agent for heavy metals,
• not more than 5% by weight of graying inhibitor,
• not more than 5% by weight of color transfer inhibitor, and
• not more than 5% by weight of foam inhibitor.

35. Oxidation dyes for dying keratinic fibers, in particular hair, containing choline oxidases according to any of claims 1 or 2.

36. A signal reagent for producing light emission in a chemiluminescence assay, comprising a choline oxidase according to any of claims 1 or 2, a choline oxidase substrate and a chemiluminescence reagent.

37. The signal reagent according to claim 36, wherein the choline oxidase substrate is selected from choline, choline derivatives and betaine aldehyde.

38. The signal reagent according to claim 36 or 37, wherein the chemi-luminescence reagent is luminol.

39. Use of a choline oxidase according to any of claims 1 or 2 for preparing betaine.

40. Use of a choline oxidase according to any of claims 1 or 2 for preparing foodstuffs and/or food components.

41. Use of a choline oxidase according to any of claims 1 or 2 for preparing animal fodder and/or animal fodder components.

## Revendications

1. Choline oxydase dont la séquence d'acides aminés correspond à la séquence d'acides aminés indiquée dans la Seq. 1,
ou bien dont la séquence d'acides aminés correspond à la séquence d'acides aminés indiquée dans la Seq. 1 à concurrence d'au moins 85 % et que l'on obtient à partir d'une choline oxydase dont la séquence d'acides aminés correspond à la séquence d'acides aminés indiquée dans la Seq. 1, via un ou plusieurs échanges conservatifs d'acides aminés.

2. Choline oxydase dont la séquence d'acides aminés correspond à une des séquences d'acides aminés indiquées dans les Seq. 10 à Seq. 15 à concurrence de 100 %.

3. Acide nucléique codant pour une choline oxydase selon l'une quelconque des revendications 1 ou 2, dont la séquence d'acides aminés contient une partie qui est identique à une des séquences d'acides aminés indiquées dans les Seq. 1 ou 10 à 15 à concurrence d'au moins 90 %, de manière particulièrement préférée à concurrence d'au moins 95 % et de manière tout particulièrement préférée à concurrence de 100 %.

4. Acide nucléique codant pour une choline oxydase selon l'une quelconque des revendications 1 ou 2, dont la séquence nucléotidique correspond à la séquence nucléotidique indiquée dans la Seq. 4 à concurrence d'au moins 85 %.

5. Acide nucléique codant pour une choline oxydase selon l'une quelconque des revendications 1 ou 2, dont la séquence nucléotidique correspond à la séquence nucléotidique indiquée dans Seq. 4 à concurrence d'au moins 90 %, de manière particulièrement préférée à concurrence d'au moins 95 % et de manière tout particulièrement préférée à concurrence de 100 %.

6. Acide nucléique codant pour une choline oxydase, dont la séquence nucléotidique correspond à une des séquences nucléotidiques indiquées dans les Seq. 17 à 22 à concurrence de 100 %.

7. Oligonucléotide comprenant une des séquences indiquées dans la Seq. 8 ou dans la Seq. 9 à utiliser comme amorce PCR.

8. Sonde d'ADN spécifique pour une choline oxydase bactérienne, dont la séquence nucléotidique correspond à la séquence nucléotidique indiquée dans Seq. 7 à concurrence d'au moins 85 %, en particulier à concurrence d'au moins 90 %, de manière particulièrement préférée à concurrence d'au moins 95 % et de manière tout particulièrement préférée à concurrence de 100 %.

9. Utilisation d'un oligonucléotide selon la revendication 7 ou d'un acide nucléique selon la revendication 8 pour l'identification et/ou l'obtention d'une choline oxydase.

10. Utilisation d'une choline oxydase selon l'une quelconque des revendications 1 ou 2, pour la fusion ou la liaison avec une autre protéine, en particulier pour le développement d'une nouvelle enzyme.

11. Utilisation d'un acide nucléique selon l'une quelconque des revendications 3 à 6, pour la fusion avec un autre acide nucléique, en particulier pour le développement d'une nouvelle enzyme.

12. Vecteur qui contient un des domaines d'acides nucléiques décrits aux revendications 3 à 6.

13. Vecteur de clonage selon la revendication 12.

14. Vecteur d'expression selon la revendication 12.

15. Cellule hôte contenant un vecteur selon l'une quelconque des revendications 12 à 14, dans laquelle la cellule hôte est un micro-organisme.

16. Cellule hôte selon la revendication 15, **caractérisé en ce qu'**il s'agit d'une bactérie, en particulier une bactérie qui sécrète la protéine formée dans le milieu environnant.

17. Cellule hôte selon la revendication 16, **caractérisée en ce qu'**elle fait partie du genre Escherichia, en particulier de l'espèce Escherichia coli.

18. Cellule hôte selon la revendication 15, **caractérisée en ce qu'**elle fait partie du genre Bacillus, de préférence de l'espèce de Bacillus licheniformis, Bacillus amyloliquefaciens, Bacillus subtilis ou Bacillus alcalophilus.

19. Cellule hôte selon la revendication 15, **caractérisée en ce qu'**elle fait partie, de manière particulièrement préférée, du genre Arthrobacter et, au sein de ce genre, de l'espèce oxidans.

20. Cellule hôte selon la revendication 15, **caractérisée en ce qu'**il s'agit d'une cellule eucaryote, en particulier une cellule eucaryote qui soumet la protéine formée à une modification posttraductionnelle.

21. Utilisation d'une choline oxydase selon l'une quelconque des revendications 1 ou 2, à titre d'agent générant du peroxyde d'hydrogène in situ.

22. Utilisation selon la revendication 21, **caractérisée en ce que** la choline oxydase présente le domaine d'acides aminés comprenant la séquence GxGxxG aux positions 20 à 25.

23. Utilisation selon la revendication 22, **caractérisée en ce que** la séquence GxGxxG représente la séquence GGGSAG.

24. Utilisation d'une choline oxydase selon la définition fournie dans l'une quelconque des revendications 21 à 23 pour la décoloration, pour l'inhibition du transfert de couleurs et pour la désinfection.

25. Produit de beauté pour le corps, shampooing, produit de soins pour les cheveux, produit de soins pour la bouche, pour les dents ou pour les prothèses dentaires, cosmétique, produit de lavage, produit de nettoyage, produit de rinçage, produit de lavage pour les mains, détergent pour la vaisselle à la main, détergent pour le lave-vaisselle, désinfectant ou produit pour le traitement de blanchiment ou de désinfection de milieux filtrants, de textiles, de fourrures, de papier, de peaux ou de cuirs, contenant une choline oxydase selon l'une quelconque des revendications 1 ou 2.

26. Agent de lavage ou de blanchiment, contenant un système de blanchiment qui est en mesure, dans les conditions d'utilisation de l'agent, de générer du peroxyde d'hydrogène, et le cas échéant, un agent tensioactif synthétique, des builders organiques et/ou inorganiques, ainsi que d'autres constituants habituels d'agents de blanchiment ou de lavage, en particulier des activateurs du blanchiment, de préférence du TEAD, **caractérisé en ce que** le système de blanchiment est constitué par une choline oxydase selon l'une quelconque des revendications 1 ou 2 et par un substrat pour la choline oxydase.

27. Agent selon la revendication 26, **caractérisé en ce que** le substrat est de la choline, un aldéhyde de bétaïne ou un dérivé de choline.

28. Agent selon la revendication 26 ou 27, **caractérisé en ce qu'**il présente une activité d'oxydase de 3 U/g à 20.000 U/g.

29. Agent selon l'une quelconque des revendications 26 à 28, **caractérisé en ce qu'**il est présent sous la forme d'une poudre versable possédant une densité apparente de 300 g/l à 1200 g/l, en particulier de 500 g/l à 900 g/l.

30. Agent selon l'une quelconque des revendications 26 à 28, **caractérisé en ce qu'**il est présent sous la forme d'un détergent pâteux ou liquide.

31. Agent selon l'une quelconque des revendications 26 à 28 ou 30, **caractérisé en ce qu'**il est présent sous la forme d'un détergent liquide non aqueux ou d'une pâte non aqueuse.

32. Agent selon l'une quelconque des revendications 26 à 28, 30 ou 31, **caractérisé en ce qu'**il est présent sous la forme d'un détergent liquide aqueux ou d'une pâte aqueuse et conditionné dans un récipient imperméable à l'air à partir duquel il est libéré peu de temps avant l'emploi ou pendant le processus de lavage.

33. Agent selon l'une quelconque des revendications 26 à 32, **caractérisé en ce que** la choline oxydase et/ou le substrat pour cette enzyme est/sont enveloppé(s) d'une substance imperméable à la température ambiante ou en l'absence d'eau pour l'enzyme et/ou son substrat, qui, dans les conditions d'utilisation de l'agent, devient perméable pour l'enzyme et/ou son substrat.

34. Agent selon l'une quelconque des revendications 26 à 33 **caractérisé en ce qu'**il contient en plus du système de blanchiment
• un agent tensioactif à concurrence de 5 % en poids à 70 % en poids, en particulier à concurrence de 10 % en poids à 50 % en poids ;
• une matière inorganique faisant office de builder soluble dans l'eau ou apte à être dispersée dans l'eau à concurrence de 10 % en poids à 65 % en poids, en particulier à concurrence de 12 % en poids à 60 % en poids ;
• des substances organiques faisant office de builder solubles dans l'eau à concurrence de 1 % en poids à 10 % en poids, en particulier à concurrence de 2 % en poids à 8 % en poids ;
• des acides inorganiques et/ou organiques solides, respectivement des sels acides, à concurrence d'une valeur qui n'est pas supérieure à 15 % en poids ;
• des agents complexants pour des métaux lourds à concurrence d'une valeur qui n'est pas supérieure à 5 % en poids ;
• un inhibiteur du grisonnement à concurrence d'une valeur qui n'est pas supérieure à 5 % en poids ;
• un inhibiteur du transfert des couleurs à concurrence d'une valeur qui n'est pas supérieure à 5 % en poids ; et
• un agent antimousse à concurrence d'une valeur qui n'est pas supérieure à 5 % en poids.

35. Colorant d'oxydation pour la teinture de fibres kératiniques, en particulier de cheveux, contenant des choline oxydases selon l'une quelconque des revendications 1 ou 2.

36. Réactif signal pour générer une émission de lumière dans un dosage basé sur la chimiluminescence, comprenant une choline oxydase selon l'une quelconque des revendications 1 ou 2, un substrat de choline oxydase et un réactif de chimiluminescence.

37. Réactif signal selon la revendication 36, dans lequel le substrat de choline oxydase est choisi parmi la choline, des dérivés de choline et un aldéhyde de bétaïne.

38. Réactif signal selon la revendication 36 ou 37, dans lequel le réactif de chimiluminescence est du luminol.

39. Utilisation d'une choline oxydase selon l'une quelconque des revendications 1 ou 2, pour la préparation de bétaïne.

40. Utilisation d'une choline oxydase selon l'une quelconque des revendications 1 ou 2, pour la préparation de denrées alimentaires et/ou de constituants de denrées alimentaires.

41. Utilisation d'une choline oxydase selon l'une quelconque des revendications 1 ou 2, pour la préparation d'aliments pour animaux et/ou de constituants d'aliments pour animaux.
